# EUROPEAN PATENT SPECIFICATION

(11) **EP 0 330 191 B1**
(45) Date of publication and mention of the grant of the patent: **02.10.1996**
(21) Application number: 89103127.0
(22) Date of filing: 23.02.1989
(51) Int. Cl.: C12N 15/00

(54) **DNA encoding CD40**
CD40 kodierende DNA
ADN encodant CD40

(30) Priority: 25.02.1988 US 160416
(43) Date of publication of application: 30.08.1989
(60) Divisional application: 96104493.0
(73) Proprietor: THE GENERAL HOSPITAL CORPORATION, Boston, MA 02114 (US)
(72) Inventor: Seed, Brian, Dr. Department of Molecular Biology, Boston, MA 02114 (US); Allen, Janet, Boston, MA 02116 (US); Aruffo, Alejandro, Belmont, MA 02178 (US); Camerini, David, Cambridge, MA 02108 (US); Lauffer, Leander, Dr., Boston, MA 02108 (US); Oquendo, Carmen Patricia, Boston, MA 02108 (US); Simmons, David, Boston, MA 02114 (US); Stamenkovic, Ivan, Brookline, MA 02146 (US); Stengelin, Siegfried, Dr., D-6238 Hofheim am Taunus (DE)
(74) Representative: Fischer, Hans-Jürgen, Dr.

(56) References cited:
- WO-A-88/00209
- PROCEEDINGS OF THE NATL. ACADEMY OF SCIENCES USA, vol. 84, no. 10, May 1987, Washington, DC (US); B. SEED et al., pp. 3365-3369
- THE EMBO JOURNAL, vol. 6, no. 11, November 1987, IRL Press Ltd., Oxford (GB); A. ARUFFO et al., pp. 3313-3316
- PROCEEDINGS OF THE NATL. ACADEMY OF SCIENCES USA, vol. 84, no. 23, December 1987, Washington, DC (US); A. ARUFFO et al., pp. 8573-8577
- NATURE, vol. 329, 29 October 1987; B. SEED, pp. 840-842
- LEUCOCYTE TYPING III, McMichael et al. (eds.), Oxford Univ. Press, 1987; pp. 306-307, 590

## Description

### Background

A basic tool in the field of recombinant genetics is the conversion of poly(A)⁺ mRNA to double-stranded (ds) cDNA, which then can be inserted into a cloning vector and expressed in an appropriate host cell. Molecular cloning methods for ds cDNA have been reviewed, for example by Williams, "The Preparation and Screening of a cDNA Clone Bank," in Williamson, ed., Genetic Engineering, Vol. 1, p. 2, Academic Press, New York (1981); Maniatis, "Recombinant DNA", in Prescott ed., Cell Biology, Academic Press, New York (1980); and Efstratiadis et al., "Cloning of Double-Stranded DNA," in Stelo et al., Genetic Engineering, Vol. 1, p. 15, Plenum Press, New York (1979).

A substantial number of variables affect the successful cloning of a particular gene and cDNA cloning strategy thus must be chosen with care. A method common to many cDNA cloning strategies involves the construction of a "cDNA library" which is a collection of cDNA clones derived from the total poly(A)⁺ mRNA derived from a cell of the organism of interest.

A mammalian cell may contain up to 30,000 different mRNA sequences, and the number of clones required to obtain low-abundance mRNAs, for example, may be much greater. Methods of constructing genomic eukaryotic DNA libraries in different expression vectors, including bacteriophage λ , cosmids, and viral vectors, are known. Some commonly used methods are described, for example, in Maniatis et al., Molecular Cloning: A Laboratory Manual, Cold Spring Harbor Laboratory, publisher, Cold Spring Harbor, New York (1982).

Once a genomic cDNA library has been constructed, it is necessary to isolate from the thousands of host cells the cell containing the particular human gene of interest. Many different methods of isolating target genes from cDNA libraries have been utilized, with varying success. These include, for example, the use of nucleic acid probes, which are labeled mRNA fragments having nucleic acid sequences complementary to the DNA sequence of the target gene. When this method is applied to cDNA clones of abundant mRNAs in transformed bacterial hosts, colonies hybridizing strongly to the probe are likely to contain the target DNA sequences. The identity of the clone then may be proven, for example, by in situ hybridization/selection (Goldberg et al., Methods Enzymol., 68:206 (1979)) hybrid-arrested translation (Paterson et al., Proceedings of the National Academy of Sciences, 74:4370 (1977)), or direct DNA sequencing (Maxam and Gilbert, Proceedings of the National Academy of Sciences, 74:560 (1977); Maat and Smith, Nucleic Acids Res., 5:4537 (1978)).

Such methods, however, have major drawbacks when the object is to clone mRNAs of relatively low abundance from cDNA libraries. For example, using direct in situ colony hybridization, it is very difficult to detect clones containing cDNA complementary to mRNA species present in the initial library population at less than one part in 200. As a result, various methods for enriching mRNA in the total population (e.g. size fractionation, use of synthetic oligodeoxynucleotides, differential hybridization, or immunopurification) have been developed and are often used when low abundance mRNAs are cloned. Such methods are described, for example, in Maniatis et al., Molecular Cloning: A Laboratory Manual, supra.

Many functional eukaryotic proteins initially exist in the form of precursor molecules which contain leader or signal sequences at their N-terminal ends. These leader sequences bind to the cell membrane and draw the remainder of the protein through the lipid bilayer, after which the signal sequence is cleaved from the protein by a signal peptidase enzyme. The protein thus functions only after secretion from the cells (for example, insulin, serum albumin, antibodies, and digestive tract enzymes), or after the proteins have been anchored to the outer surface of a cell membrane (for example, histocompatibility antigens).

The cell surface antigens characteristic of mammalian T lymphocytes are additional examples of proteins that anchor to the cell surface. In mammals, certain cells derived from bone marrow mature into lymphocytes, which are present in the lymphoid organs, including the thymus, spleen, lymph nodes, and lymphoid aggregates, and also circulate actively through the blood and lymph systems. Mature lymphocyte cells may be divided into two populations: thymus-dependent (T) lymphocytes and thymus-independent (B) lymphocytes. T lymphocytes migrate to the interior of the thymus, where they undergo differentiative proliferation. During their differentiation process, they express characteristic cell surface membrane alloantigens, including Thy-1, TLA, gv-1, Ly-1, Ly-2, Ly-3, and Ly-5. As they mature, T lymphocytes lose the TLA antigens and some of the Thy-1 antigens, and gain histocompatibility antigens, acquiring the membrane conformation typical of the recirculating T lymphocytes. This is described, for example, by Mota, "Activity of Immune Cells," in Bier et al., eds., Fundamentals of Immunology, 2d Ed., Springer-Verlag, Berlin, pp. 35-62 (1986).

T lymphocytes are involved indirectly in the formation of antibodies and their activities thus have required complex analysis of cell function, rather than simple antibody titer measurement. Partly due to this, their importance in development of immunologic competence was not recognized until relatively recently. Mature T lymphocytes synthesize and express an unique pattern of surface glycoprotein antigens which serve as markers for identification of different T lymphocyte subpopulations, including T helper cells, T suppressor cells, and T cytotoxic cells. Each of these subpopulations plays a very important role in regulating the immune system. (Mota, supra).

In humans, the functional and phenotypic heterogeneity of T lymphocytes is well accepted. Two major subpopulations are known: effector T cells mediating cellular immunity; and regulator T cells containing helper and suppressor T lymphocytes. These two subpopulations have been defined with heteroantisera, autoantibodies, and monoclonal antibodies directed at cell surface antigens. For example, earlier in their development, human lymphoid cells in the thymus express an antigen designated T11 which reacts strongly to a monoclonal antibody designated Cluster of Differentiation 2 (CD2), and react slightly with monoclonal antibody CD5 to cell surface antigen T1. During maturation, these cells lose T11 (CD2) and acquire three new antigens defined by monoclonal antibodies CD4, CD8, and CD1. With further maturation, the thymocytes cease to express cell surface antigens reactive with monoclonal antibody CD1, express the T3 antigen reactive with monoclonal antibody CD3, and then segregate into two subpopulations which express either T4 (CD4) or T8 (CD8) antigen. Immunologic competence is acquired at this stage, but is not completely developed until thymic lymphocytes migrate outside the thymus. (Mota, supra.) In contrast with the majority of thymocytes, circulating T lymphocytes express the T1 (CD5) and T3 (CD3) antigens. The T4 (CD4) antigen is present on approximately 55-65% of peripheral T lymphocytes, whereas the T8 (CD8) antigen is expressed on 20-30%. These two subpopulations correspond to helper and to suppressor and cytotoxic T cells, respectively.

In addition to providing a convenient means of distinguishing T lymphocyte subpopulations, these cell surface antigens are important for mature T cell activation and effector function. T cell activation involves a complex series of cell surface interactions between the T cell and the target cell or stimulator cell in addition to binding of the T cell receptor to its specific antigen.

For example, CD2, the human T cell erythrocyte receptor, allows thymocytes and T-lymphocytes to adhere to target cells (e.g., erythrocytes) and to thymic epithelium. This occurs via a specific molecular ligand for CD2, designated LFA-3, in humans, which is a widely distributed surface antigen. This phenomenon has long been employed to detect, assay and purify human cells producing antibodies to sheep erythrocytes and serves as the basis for the E-rosette test, first described by Zaalberg, Nature, 202:1231 (1964). CD2/LFA-3 interactions also have been shown to mediate cytolytic target conjugation (Shaw et al., Nature, 323:262-264 (1986), and the mixed lymphocyte reaction (Martin et al., J. Immunol., 131:180-185 (1983). Anti-CD2 monoclonal antibodies can directly activate peripheral T-lymphocytes via an antigen-independent pathway (Meuer et al., Cell, 36:897-906 (1984), indicating an even wider immunoregulatory role for CD2.

Recognition that T lymphocytes are the main effectors of cell-mediated immunity and also are involved as helper or suppressor cells in modulating the immune response has resulted in a significant contribution to the increasing practical application of clinical immunology to medicine. The scope of this application includes defense against infections, prevention of diseases by immunization, organ transplantation, blood banking, deficiencies of the immune system, and a variety of disorders that are mediated by immunologic mechanisms. Moreover, immunologic techniques frequently are used in the clinical laboratory, as in the measurement of hormones and drugs. Clinical immunology is described, for example, in Weir, ed., Handbook of Experimental Immunology in Four Volumes: Volume 4: Applications of Immunological Methods in Biomedical Sciences, 4th Ed., Blackwell Scientific Publications, Oxford (1986); Boguslaski et al., eds., Clinical Immunochemistry: Principles of Methods and Applications, Little, Brown & Co., Boston (1984); Holborow et al., eds., Immunology in Medicine: A Comprehensive Guide to Clinical Immunology, 2d Ed., Grune & Stratton, London (1983); and Petersdorf et al., eds., Harrison's Principles of Internal Medicine, 10th ed., McGraw-Hill, New York, publisher, pp. 344-391 (1983). Clearly, a more thorough understanding of the proteins which mediate the immune system would be of significant value in clinical immunology.

Use of mammalian expression libraries to isolate cDNAs encoding mammalian proteins such as those described above would offer several advantages. For example, the protein expressed in a mammalian host cell should be functional and should undergo any normal posttranslational modification. A protein ordinarily transported through the intracellular membrane system to the cell surface should undergo the complete transport process. A mammalian expression system also would allow the study of intracellular transport mechanisms and of the mechanism that insert and anchor cell surface proteins to membranes.

One common mammalian host cell, called a "COS" cell, is formed by infecting monkey kidney cells with a mutant viral vector, designated simian virus strain 40 (SV40), which has functional early and late genes, but lacks a functional origin of replication. In COS cells, any foreign DNA cloned on a vector containing the SV40 origin of replication will replicate because SV40 T antigen is present in COS cells. The foreign DNA will replicate transiently, independently of the cellular DNA.

With the exception of some recent lymphokine cDNAs isolated by expression in COS cells (Wong, G.G., et al., Science, 228:810-815 (1985); Lee, F. et al., Proceedings of the National Academy of Sciences, USA, 83:2061-2065 (1986); Yokota, T., et al., Proceedings of the National Academy of Sciences, USA, 83:5894-5898 (1986); Yang, Y., et al., Cell, 47:3-10 (1986)), however, few cDNAs in general are isolated from mammalian expression libraries. There appear to be two principal reasons for this: First, the existing technology (Okayama, H. et al., Mol. Cell. Biol., 2:161-170 (1982)) for construction of large plasmid libraries is difficult to master, and library size rarely approaches that accessible by phage cloning techniques. (Huynh, T. et al., In: DNA Cloning Vol. I, A Practical Approach, Glover, D.M. (ed.), IRL Press, Oxford (1985), pp. 49-78). Second, the existing vectors are, with one exception (Wong G.G., et al., Science, 228:810-815 (1985)), poorly adapted for high level expression, particularly in COS cells. The reported successes with lymphokine cDNAs do not imply a general fitness of the methods used, since these cDNAs are particularly easy to isolate from expression libraries. Lymphokine bioassays are very sensitive ((Wong, G.G., et al., Science, 228:810-815 (1985); Lee, F. et al., Proceedings of the National Academy of Sciences, USA, 83:2061-2065 (1986); Yokota T. et al., Proceedings of the National Academy of Sciences, USA, 83:5894-5898 (1986); Yang, Y. et al., Cell, 47:3-10 (1986)) and the mRNAs are typically both abundant and short (Wong, G.G. et al., Science, 228:810-815 (1985); Lee, F., et al., Proceedings of the National Academy of Sciences, USA, 83:2061-2065 (1986); Yokota, T., et al., Proceedings of the National Academy of Sciences, USA, 83:5894-5898 (1986); Yang, Y., et al., Cell, 47:3-10 (1986)).

Thus, expression in mammalian hosts previously has been most frequently employed solely as a means of verifying the identity of the protein encoded by a gene isolated by more traditional cloning methods. For example, Stuve et al., J. Virol., 61(2):327-335 (1987), cloned the gene for glycoprotein gB2 of herpes simplex type II strain 333 by plaque hybridization of M13-based recombinant phage vectors used to transform competent E. coli JM101. The identity of the protein encoded by the clone thus isolated was verified by transfection of mammalian COS and Chinese hamster ovary (CHO) cells. Expression was demonstrated by immunofluorescence and radioimmunoprecipitation.

Oshima et al. used plaque hybridization to screen a phase lambdagt11 cDNA library for the gene encoding human placental beta-glucuronidase. Oshima et al., Proceedings of the National Academy of Sciences, U.S.A., 84:685-689 (1987). The identity of isolated cDNA clones was verified by immunoprecipitation of the protein expressed by COS-7 cells transfected with cloned inserts using the SV40 late promoter.

Transient expression in mammalian cells has been employed as a means of confirming the identity of genes previously isolated by other screening methods. Gerald et al., Journal of General Virology, 67:2695-2703(1986). Mackenzie, Journal of Biological Chemistry, 261:14112-14117 (1986); Seif et al., Gene, 43:1111-1121 (1986); Orkin et al., Molecular and Cellular Biology, 5(4):762-767 (1985). These methods often are inefficient and tedious and require multiple rounds of screening to identify full-length or overlapping clones. Prior screening methods based upon expression of fusion proteins are inefficient and require large quantities of monoclonal antibodies. Such drawbacks are compounded by use of inefficient expression vectors, which result in protein expression levels that are inadequate to enable efficient selection.

### Summary of the invention

By means of the cloning method of the present invention, isolation and molecular cloning of the cell surface antigen CD40, has been accomplished . The nucleotide sequence of the gene cloned by the method of the present invention has been determined and the amino acid sequence of the encoded protein has been identified. The cloned gene CD40, is also the subject of the present invention.

Once the gene encoding an antigen has been cloned according to the method of the present invention, that gene can be expressed in a prokaryotic or a eukaryotic host cell to produce the encoded protein or portion thereof in substantially pure form such as it does not exist in nature. Another aspect of the present invention relates to the substantially pure cell surface antigen. The primary amino acid sequences of the CD40, antigen has been determined. The invention thus also relates to the amino acid sequences of the antigen and to the nucleotide sequences encoding the antigen.

The purified gene and protein of the present invention is useful for immunodiagnostic and immunotherapeutic applications, including the diagnosis and treatment of immune-mediated infections, diseases, and disorders in animals, including humans. It can also be used to identify, isolate and purify other antibodies and antigens. Such diagnostic and therapeutic uses comprise yet another aspect of the present invention. Moreover, the substantially pure protein of the present invention may be prepared as medicament or pharmaceutical composition for therapeutic administration. The present invention further relates to such medicaments and compositions.

### Brief Description of the Drawings

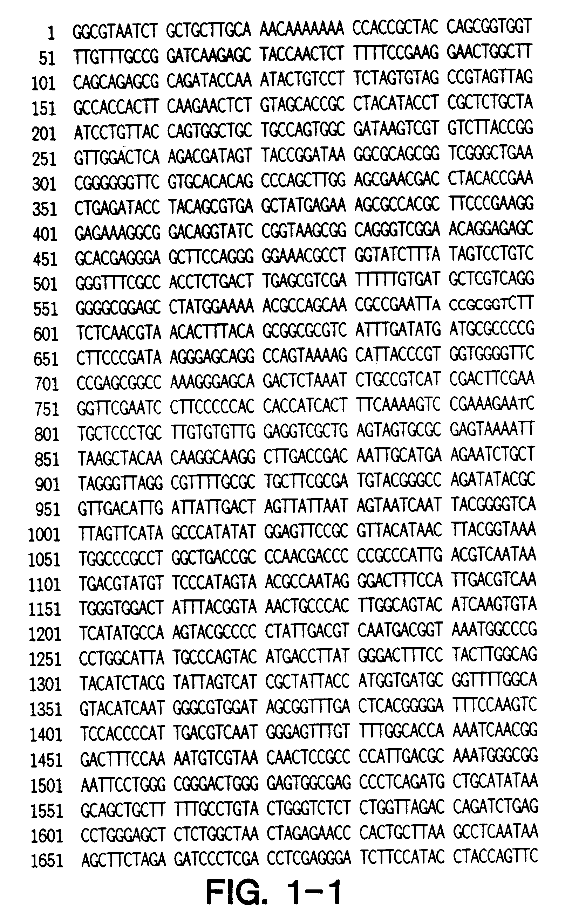

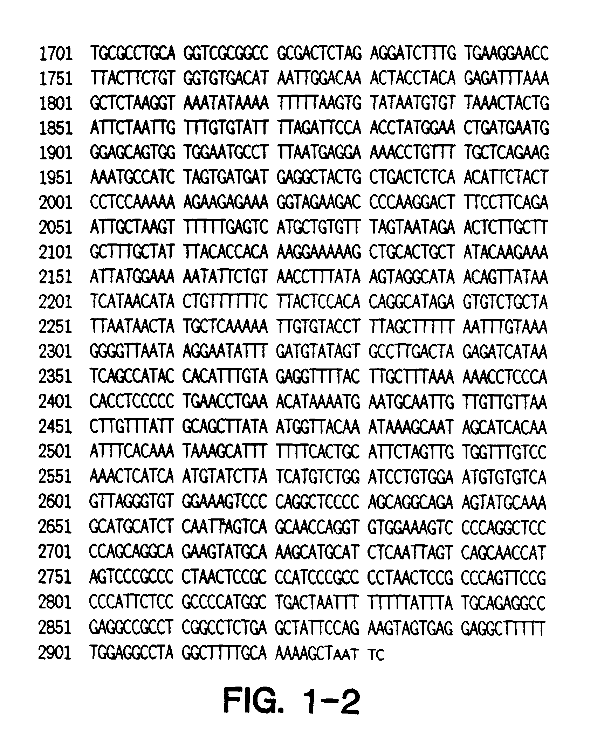

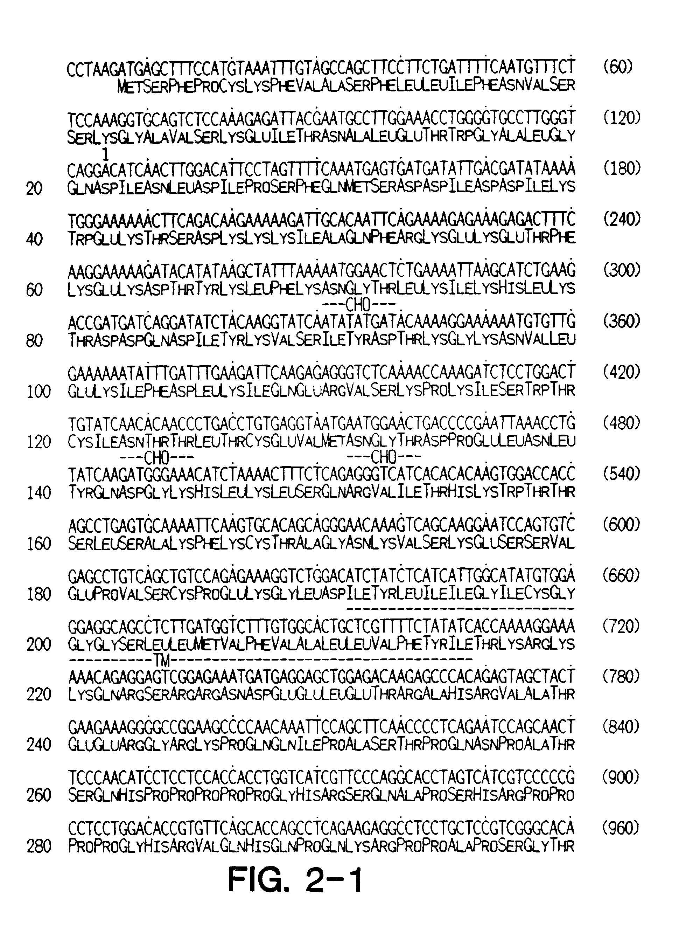

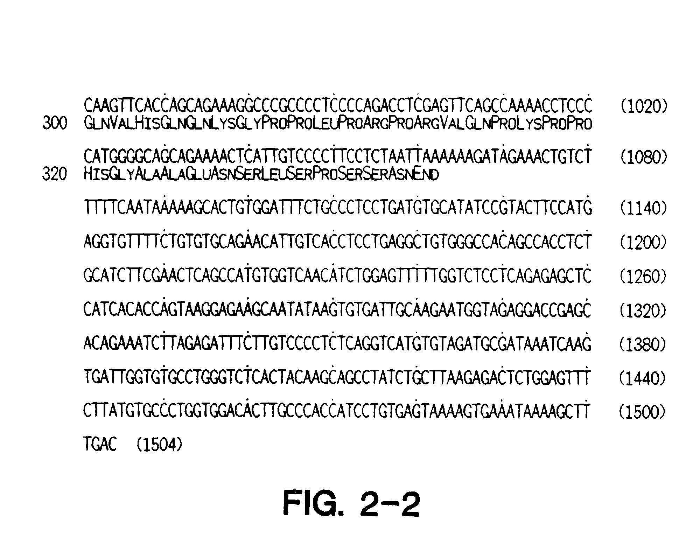

Figure 1. Nucleotide sequence of expression vector piH3
   Nucleotides 1-589 are derived from pMB1 origin (pBR322 ori); nucleotides 590-597 are derived from the SacII linker (ACCGCGT); nucleotides 598-799 are derived from the synthetic tyrosine suppressor tRNA gene (supF gene); nucleotides 800-947 are derived from a remnant of the ASV LTR fragment (PvuII to MIu1); nucleotides 948-1500 are derived from the human cytomegalovirus AD169 enhancer; nucleotides 1501-1650 are derived from HIV TATA and tat-responsive elements; nucleotides 1651-1716 are derived from the piLNXAN polylinker (HindIII to Xba); nucleotides 1717-2569 are derived from pSV to splice and poly-Addition signals; nucleotides 2570-2917 are derived from the SV40 origin of replication (pvuII to (HindIII) ; and nucleotides 2918-2922 are derived from piVX, remnant of R1 site from polylinker.

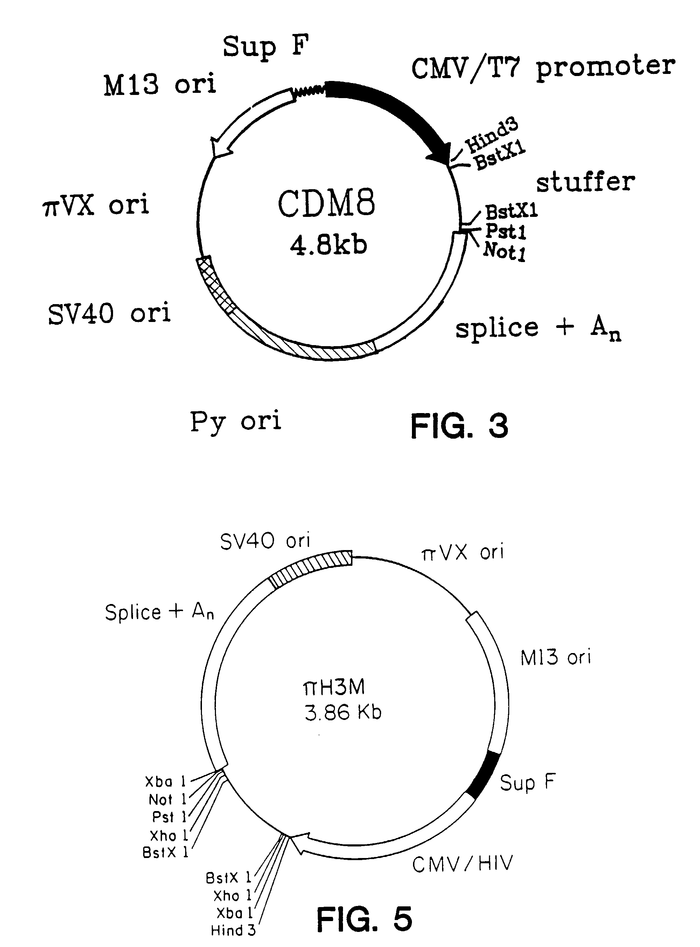

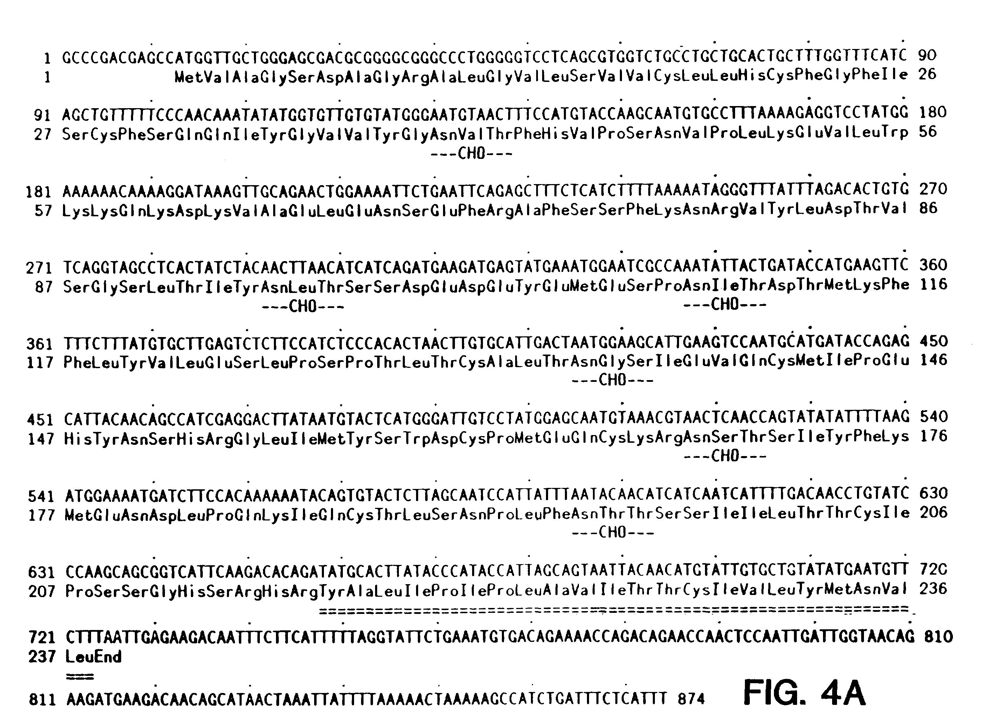

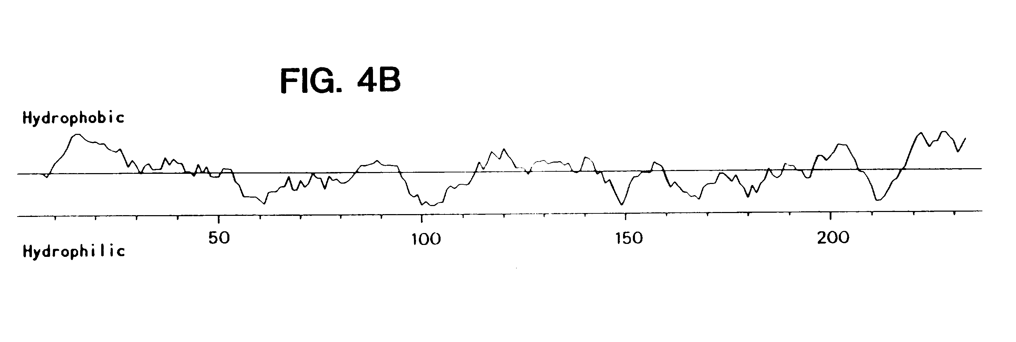

Figure 3. Restriction map of the CDM8 expression vector
   The CDM8 vector includes a deleted version of a mutant polyoma virus early region selected for high efficiency expression in both murine and monkey cells. Substantially all of the human immunodeficiency promoter region has been replaced with the cognate sequences of the human cytomegalovirus immediate early promoter, and by inclusion of a bacteriophage T7 promoter between the eukaryotic promoter and the site of cDNA insertion. Arrows indicate the direction of transcription.
Figure 5. Restriction Map of the piH3M vector
   The direction of transcription is indicated by an arrow. Restriction endonuclease sites flanking the BstXI cloning sites are shown.

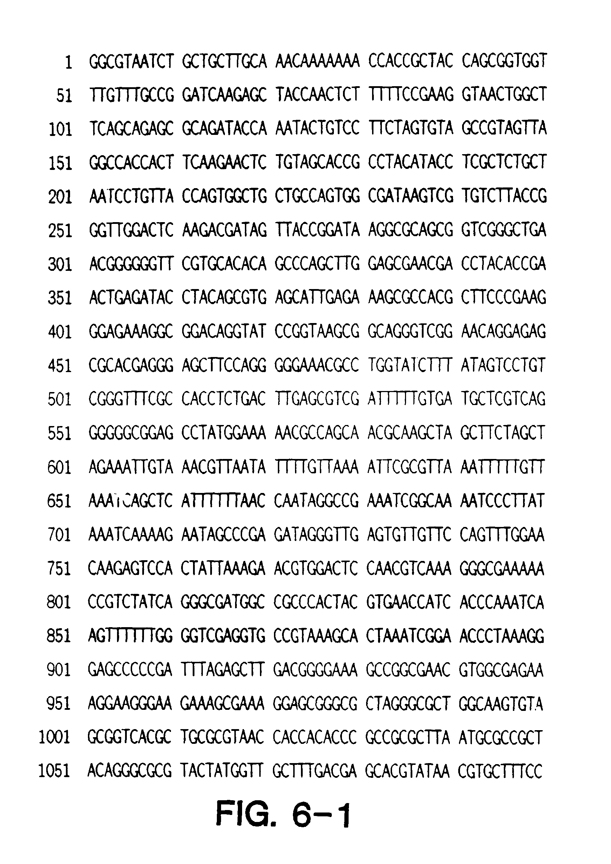

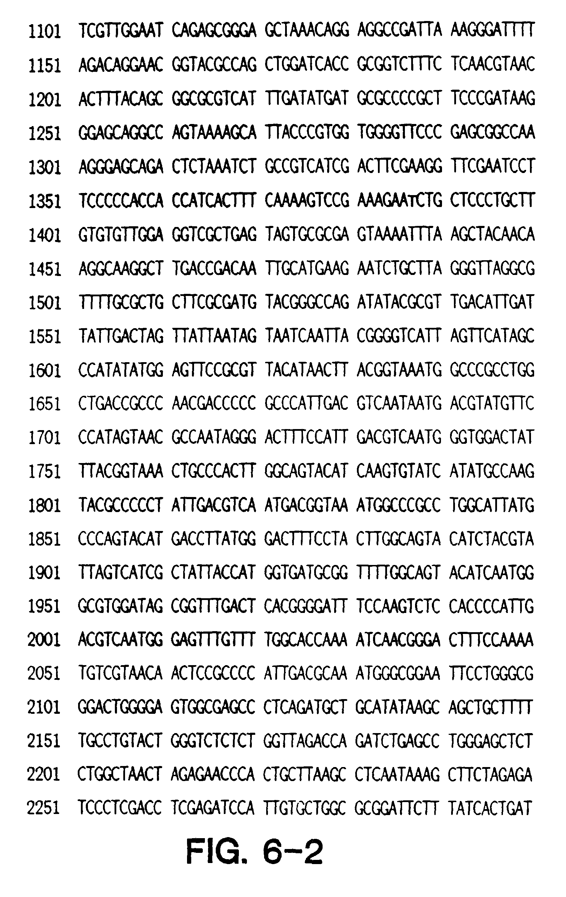

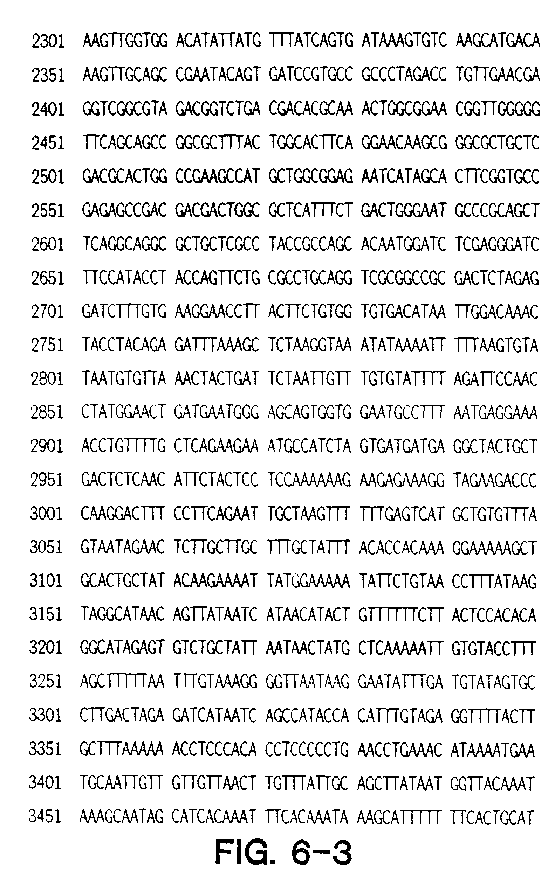

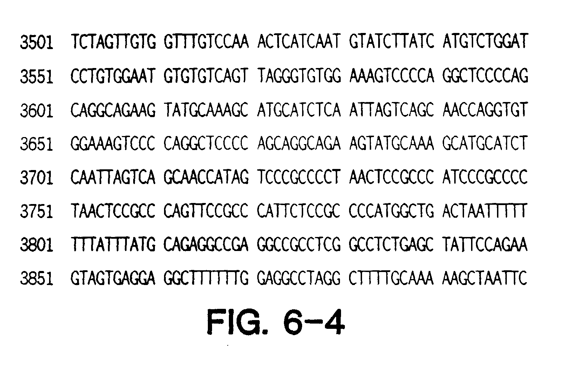

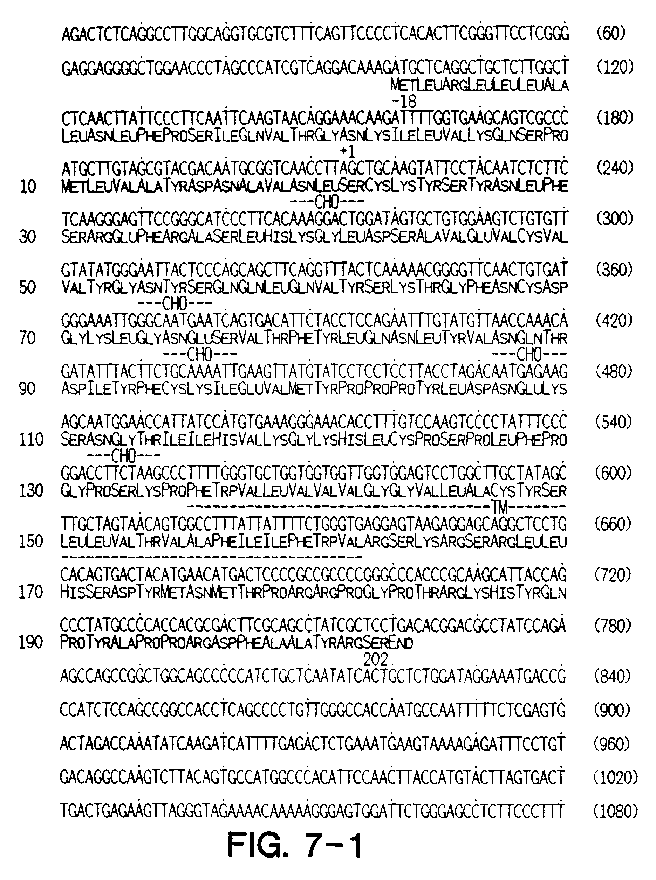

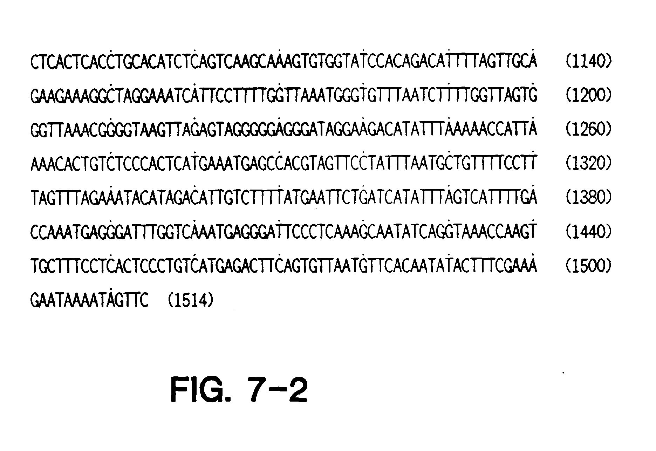

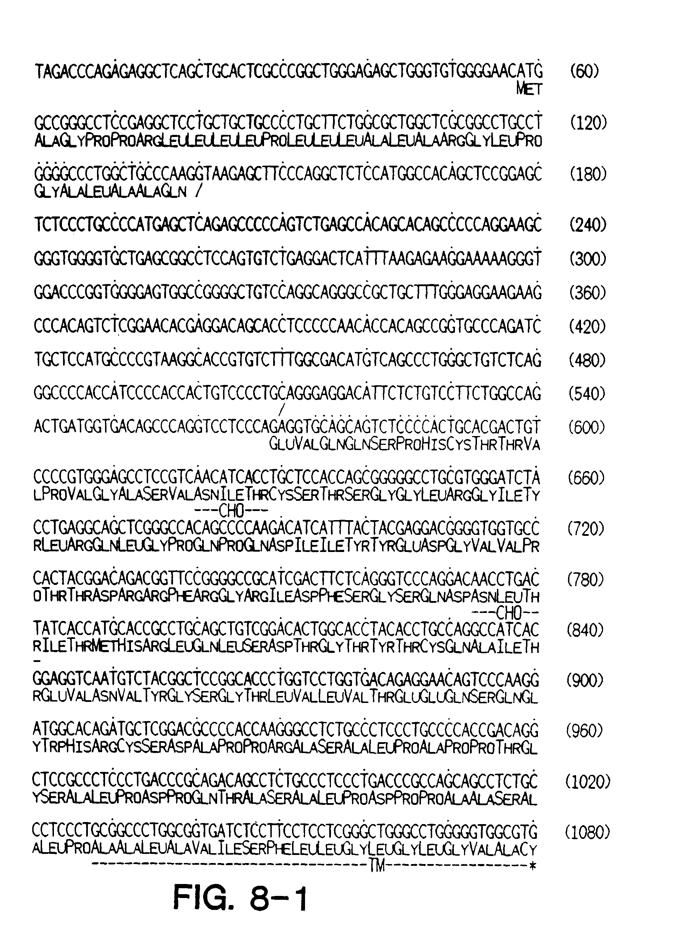

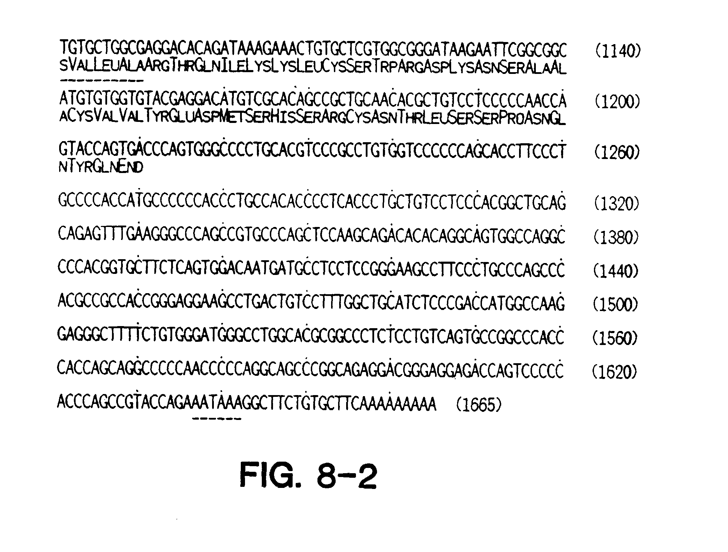

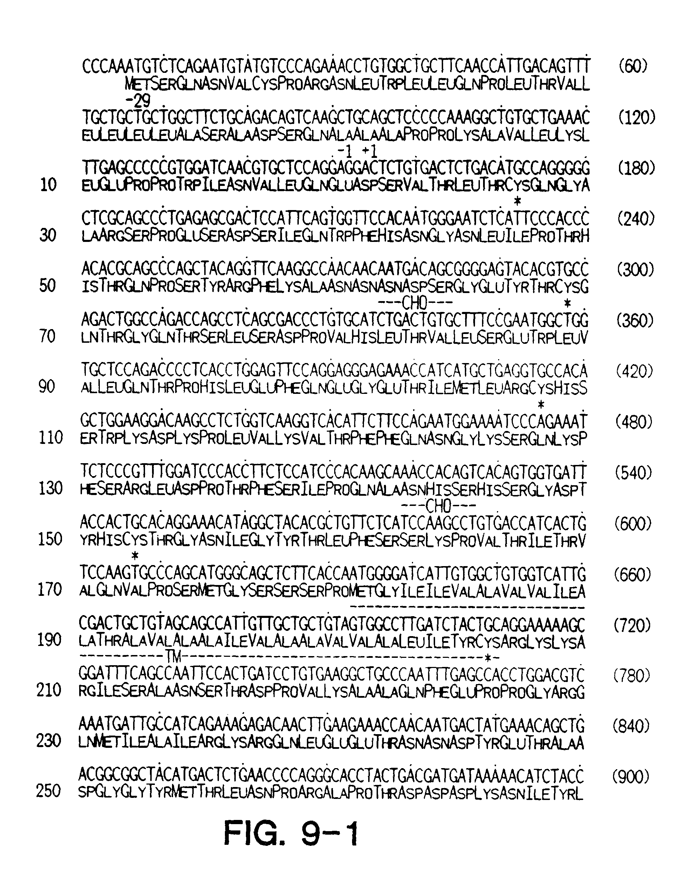

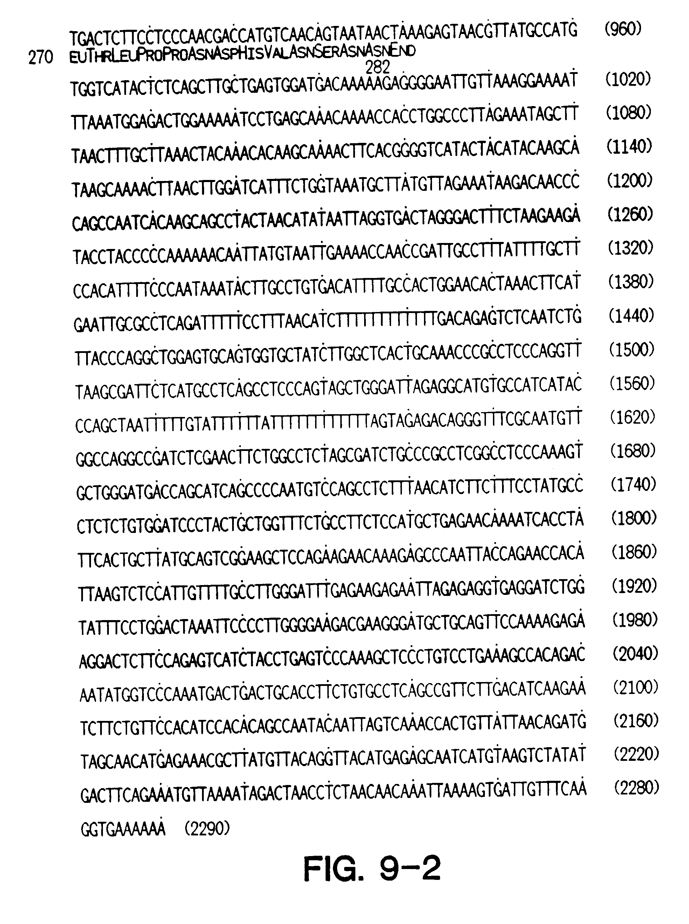

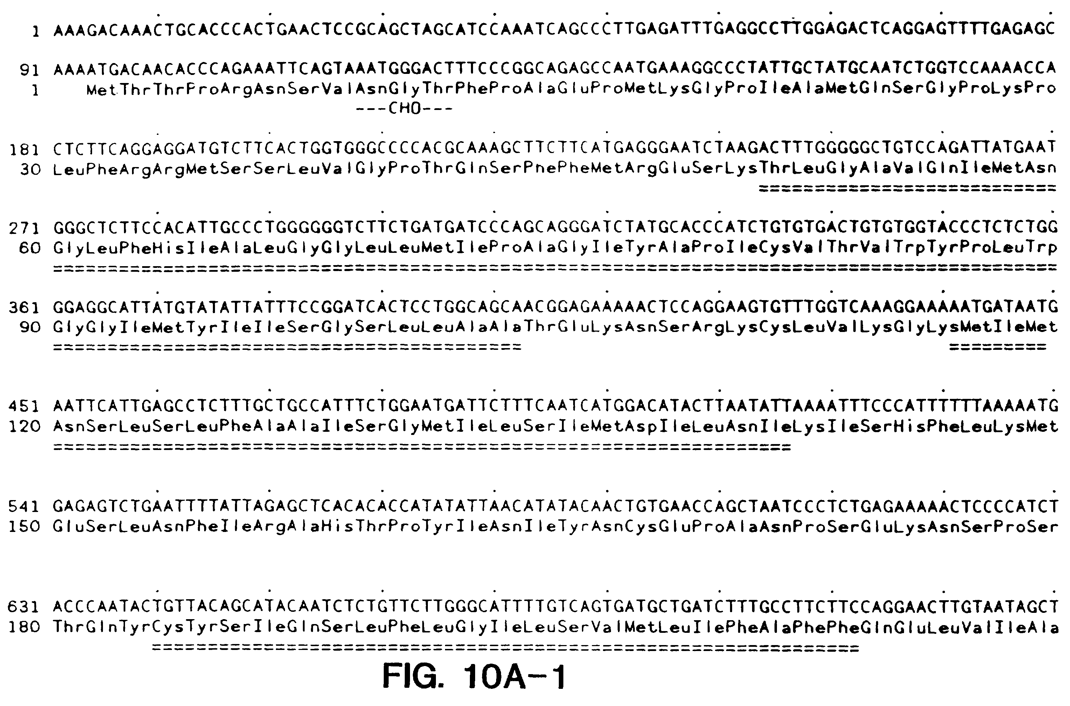

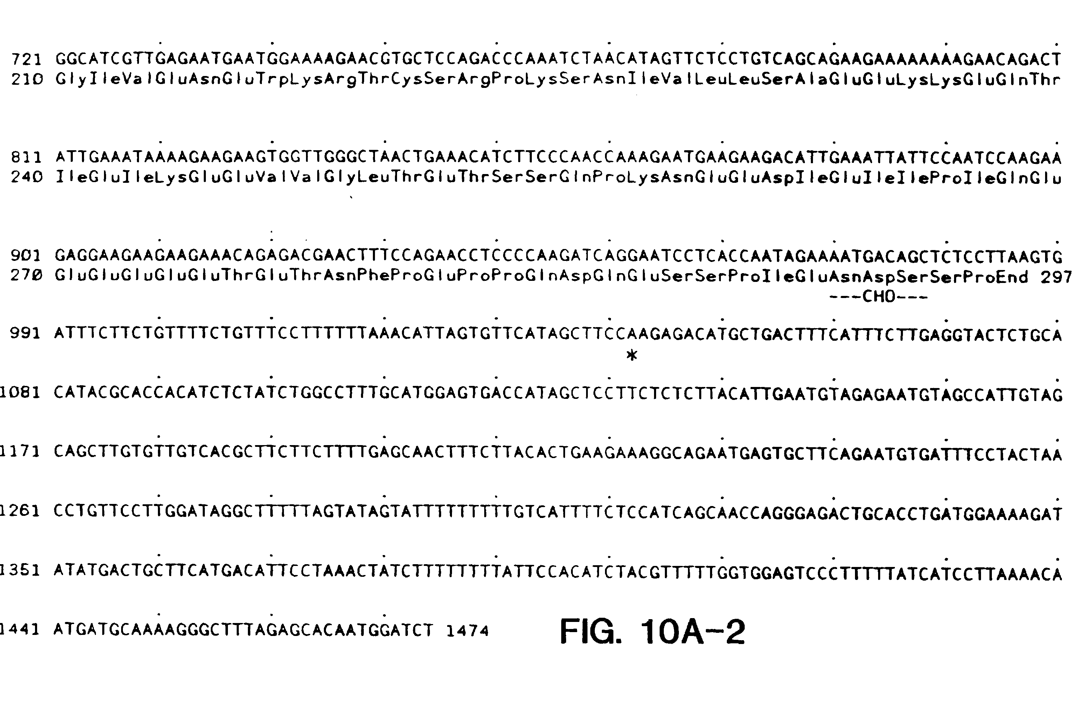

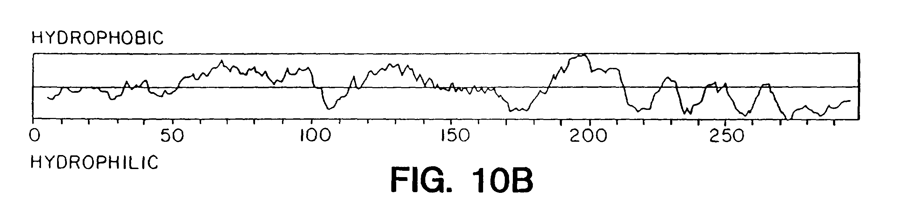

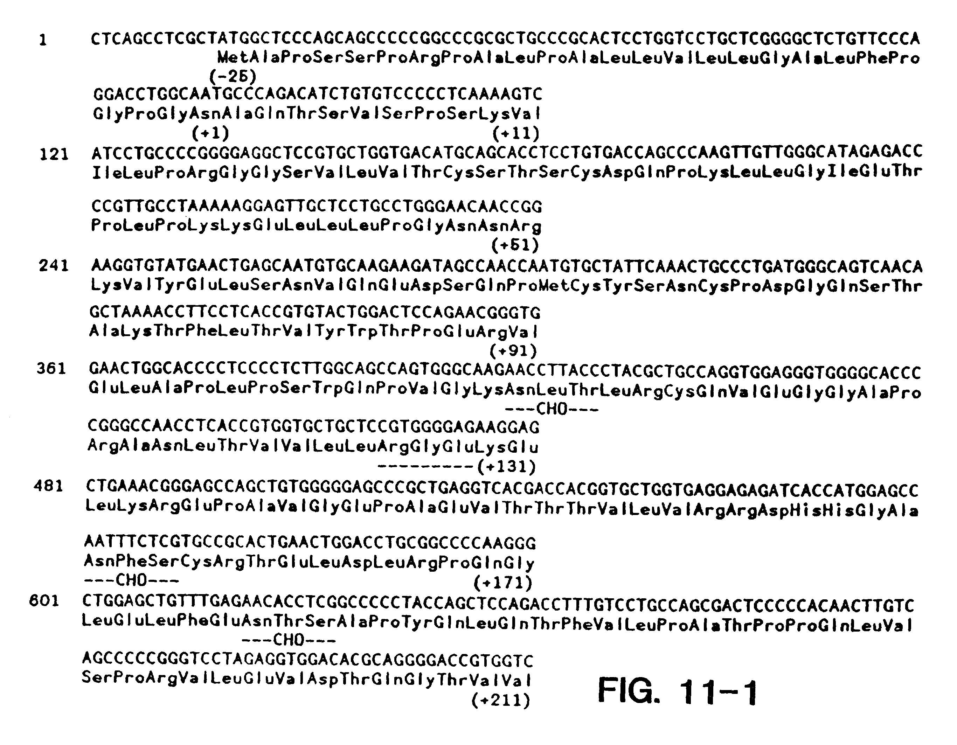

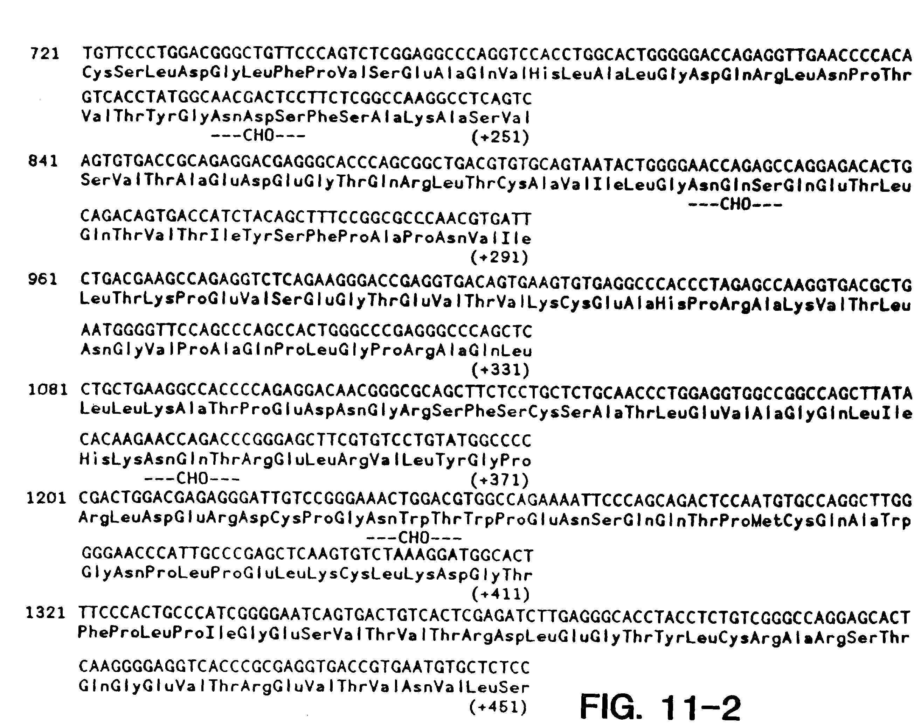

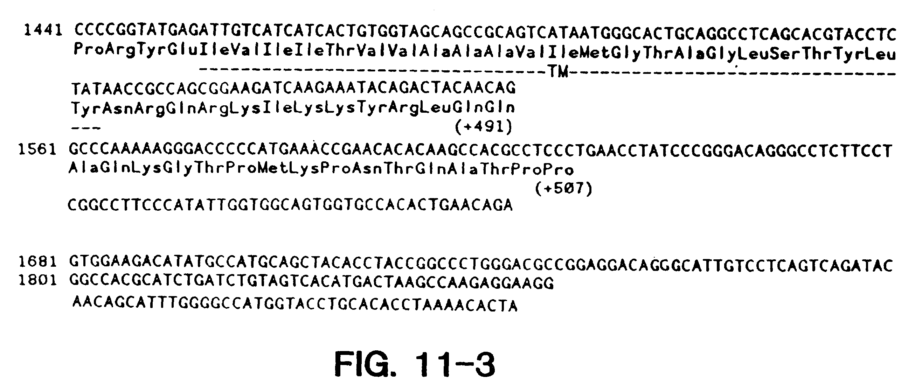

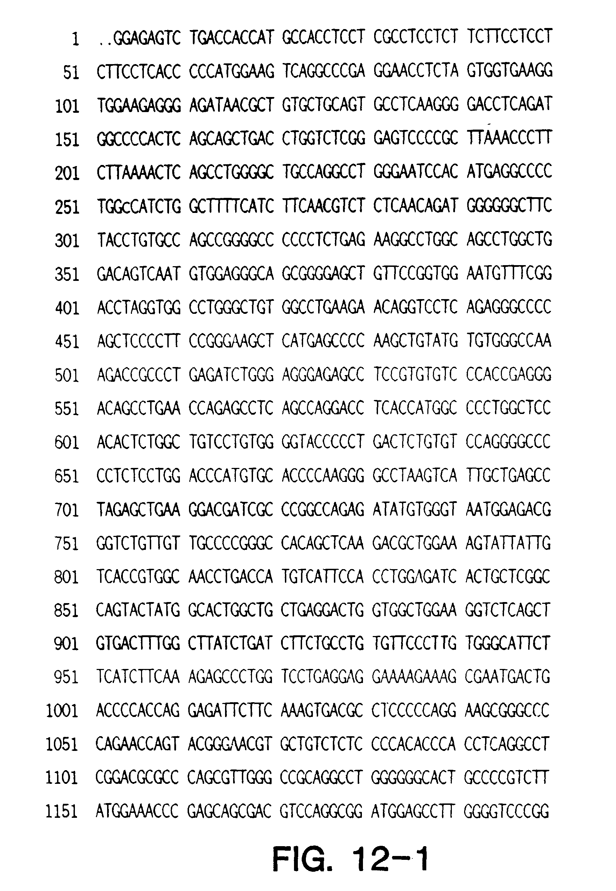

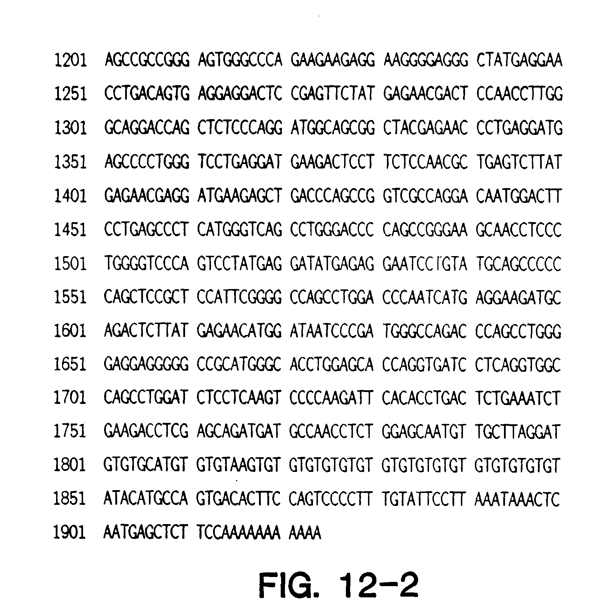

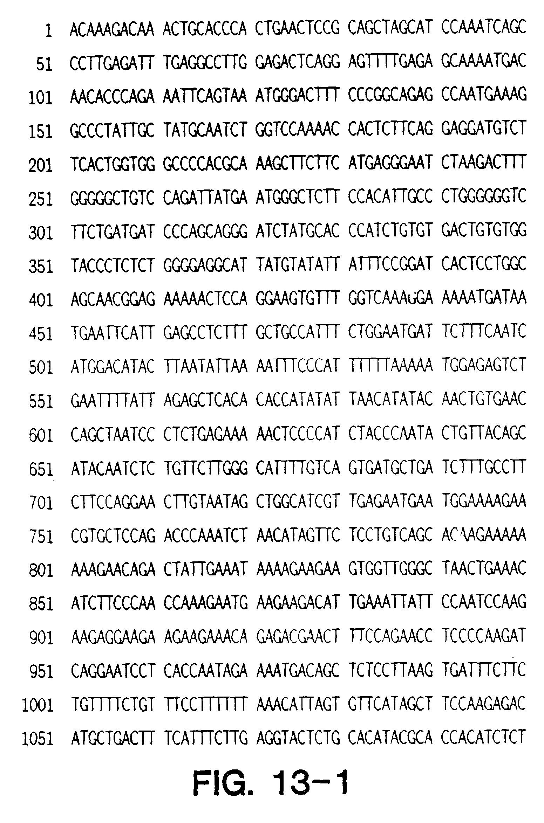

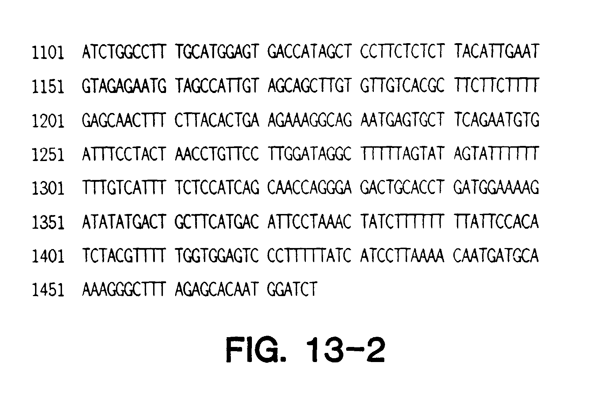

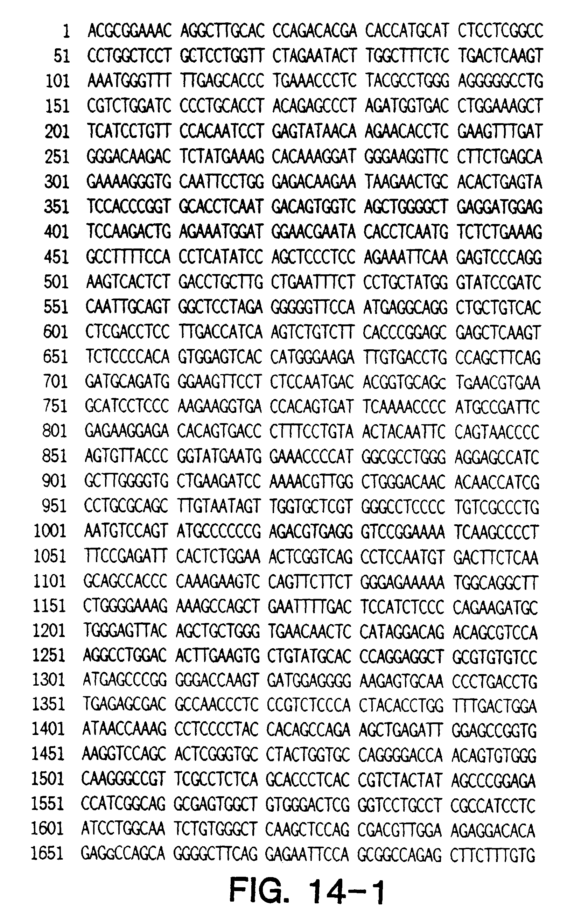

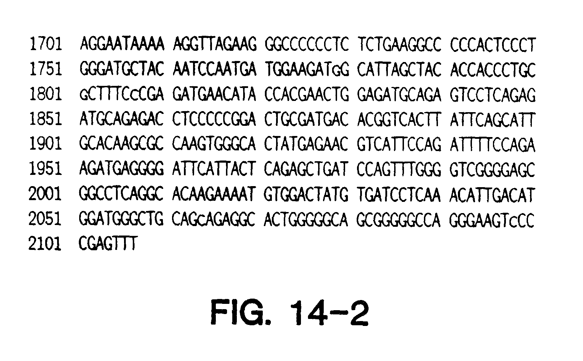

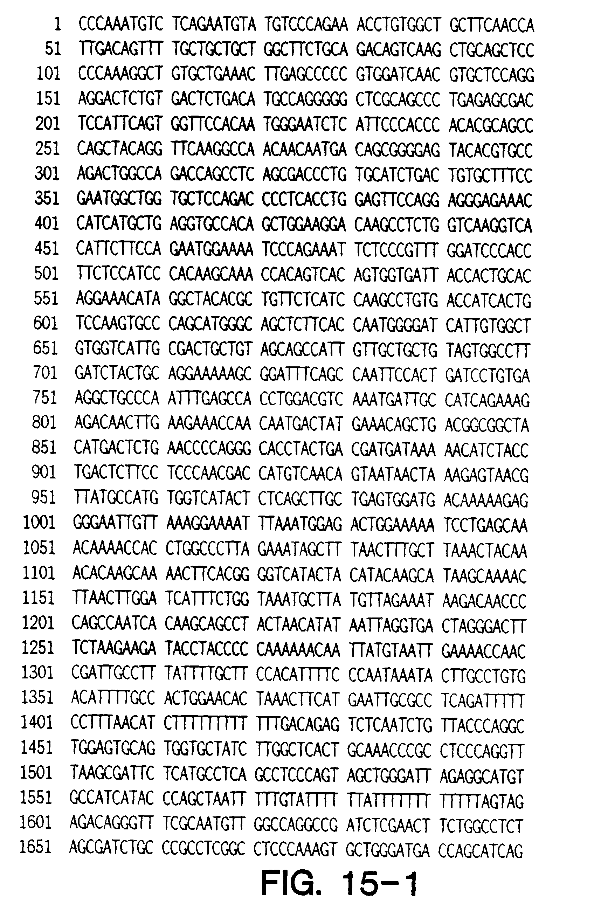

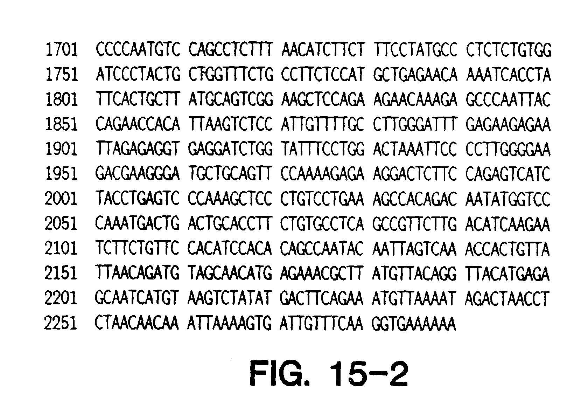

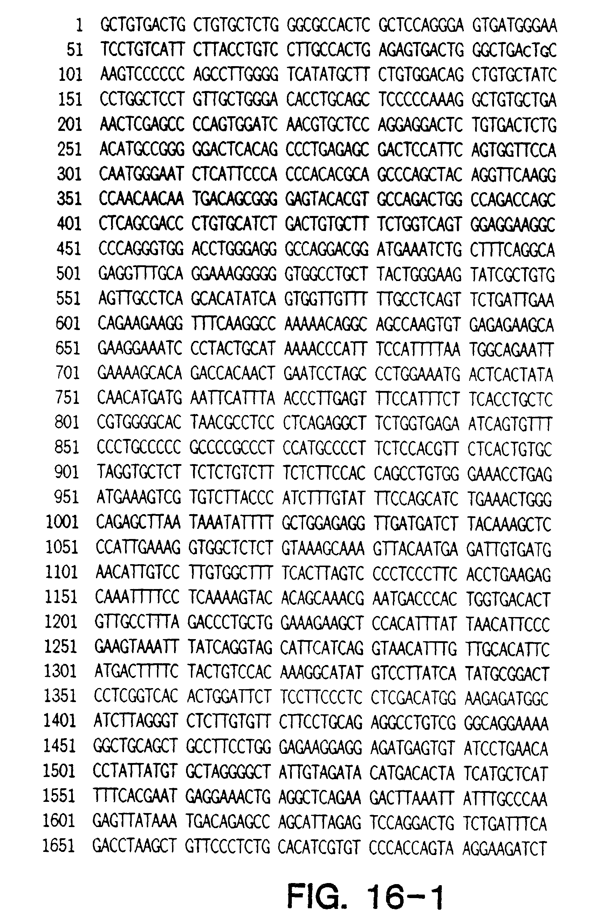

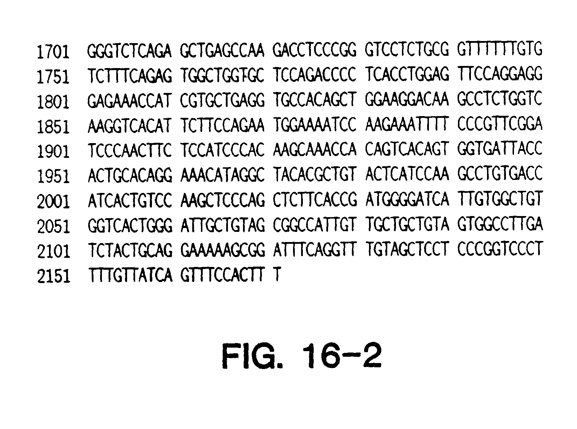

Figure 6. Nucleotide sequence of the piH3M vector
   There are 7 segments. Residues 1-587 are from the pBR322 origin of replication, 588-1182 from the M13 origin, 1183-1384 from the supF gene, 1385-2238 are from the chimeric cytomegalovirus/human immunodeficiency virus promoter, 2239-2647 are from the replaceable fragment, 2648-3547 from plasmid pSV2 (splice and polyadenylation signals), and 3548-3900 from the SV40 virus origin.

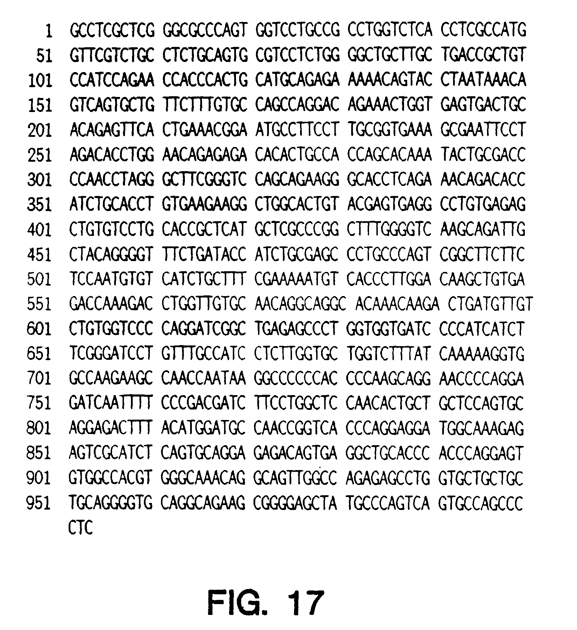

Figure 17. Nucleotide sequence of CD40

### Detailed Description of the Invention

This invention relates to a novel method for cloning cDNA encoding a cell surface antigen and to a method of constructing cDNA libraries. It also relates to particular cDNA expression vectors and components thereof, nucleotide sequences or genes isolated by the method, substantially pure cell surface antigens encoded by the cDNA segments, and methods of using the isolated nucleotide sequences and encoded products.

In the following description, reference will be made to various methodologies known to those of skill in the art of recombinant genetics. Publications and other materials setting forth such known methodologies to which reference is made are incorporated herein by reference in their entireties.

Standard reference works setting forth the general principles of recombinant DNA technology include Darnell, J.E. et al., Molecular Cell Biology, Scientific American Books, Inc., publisher, New York, N.Y. (1986); Lewin, B.M., Genes II, John Wiley & Sons, publisher, New York, N.Y. (1985); Old, R.W. et al., Principles of Gene Manipulation: An Introduction to Genetic Engineering, 2d edition, University of California Press, Berkeley, CA (1981); and Maniatis, T. et al., Molecular Cloning: A Laboratory Manual, Cold Spring Harbor, NY (1982).

By "cloning" is meant the use of in vitro recombination techniques to insert a particular gene or other DNA sequence into a vector molecule. In order to successfully clone a desired gene, it is necessary to employ methods for generating DNA fragments, for joining the fragments to vector molecules, for introducing the composite DNA molecule into a host cell in which it can replicate, and for selecting the clone having the target gene from amongst the recipient host cells.

By "cDNA" is meant complementary or copy DNA produced from an RNA template by the action of RNA-dependent DNA polymerase (reverse transcriptase). Thus a "cDNA clone" means a duplex DNA sequence complementary to an RNA molecule of interest, carried in a cloning vector.

By "cDNA library" is meant a collection of recombinant DNA molecules containing cDNA inserts which together comprise the entire genome of an organism. Such a cDNA library may be prepared by art-recognized methods described, for example, in Maniatis et al., Molecular Cloning: A Laboratory Manual, supra. Generally, RNA is first isolated from the cells of an organism from whose genome it is desired to clone a particular gene. Preferred for the purposes of the present invention are mammalian, and particularly human, cell lines. More preferred are the human tumor cell line HPB-ALL and the human lymphoblastoid cell line JY. Alternatively, RNA can be isolated from a tumor cell, derived from an animal tumor, and preferably from a human tumor. Thus, a library may be prepared from, for example, a human adrenal tumor, but any tumor may be used.

The immunoselection cloning method of the present invention comprises the preparation of a cDNA library by extracting total RNA including a particular gene from a cell, synthesizing a series of complementary double-stranded cDNA fragments from the RNA and introducing these cDNA fragments into mammalian cells in tissue culture. The mammalian cells are maintained under conditions which allow them to express the protein (i.e. the cell surface antigen). The resulting cells are exposed to a first antibody or pool (group) of antibodies directed against the cell surface antigen. This results in formation of a cell surface antigen-first antibody complex. The complexes are exposed to a substrate to which is coated or bound a second antibody directed against the first antibody. Cells expressing the cell surface antigen adhere to the substrate (because of formation of a cell surface antigen-first antibody-second antibody complex). Adherent cells are separated from non-adherent cells.

### Isolation of total RNA

The guanidium thiocyanate/CsCl method of isolating total RNA is preferred. More preferred is a guanidium thiocyanate/LiCl variant of the GuSCN/CsCl method, which has added capacity and speed. Briefly, for each ml of mix desired, 0.5g GuSCN are dissolved in 0.58ml of 25% LiCl (stock filtered through 0.45 micron filter) and 20ul of mercaptoethanol is added. Cells are spun out and the pellet is dispersed on walls by flicking, add 1ml of solution to up to 5 x 10⁷ cells. The resulting combination is sheared by polytron until nonviscous. For small scale preps (less than 10⁸ cells) layer 2ml of sheared mix on 1.5ml of 5.7M CsCl (RNase free; 1.26 g CsCl added to every ml of 10mM EDTA pH 8), overlay with RNase-free water and spin SW55 50krpm 2h. For large scale preps, layer 25ml of 12ml CsCl in a SW28 tube, overlay, and spin 24k rpm 8h. Aspirate contents carefully with a sterile pasteur pipet connected to a vacuum flask. Once past the CsCl interface, scratch a band around the tube with the pipet tip to prevent the layer on the wall of the tube from creeping down. The remaining CsCl solution is aspirated. The pellets are taken up in water (do not try to redissolve). 1/10 vol. NaOAc and 3 vol. EtOH are added and the resulting combination is spun. If necessary, the pellet is resuspended in water (e.g., at 70°). Adjust concentration to 1mg/ml and freeze. Small RNA (e.g. 5S) does not come down. For small amounts of cells, scale down volumes and overlay GuSCN with RNase-free water on gradient (precipitation is inefficient when RNA is dilute).

### Preparation of poly A⁺ RNA

Next, polyA⁺ RNA may be prepared, preferably by the oligo dT selection method. Briefly, a disposable polypropylene column is prepared by washing with 5M NaOH and then rinsing with RNase-free water. For each milligram total RNA about 0.3 ml (final packed bed) oligo dT cellulose is used. Oligo dT cellulose is prepared by resuspending about 0.5 ml of dry powder in 1 ml of 0.1M NaOH and transferring it into the column, or by percolating 0.1 NaOH through a previously used column (columns can be reused many times). This is washed with several column volumes of RNase-free water, until pH is neutral, and rinsed with 2-3 ml of loading buffer. The column bed is then removed into a sterile 15ml tube using 4-6 ml of loading buffer. The total RNA to 70°C for 2-3 min., LiCl from RNase-free stock is added (to 0.5M), and combined with oligo dT cellulose in a 15 ml tube. This is followed by vortexing or agitation for 10 min. The result is poured into a column and washed with 3 ml loading buffer and then 3 ml of middle wash buffer. mRNA is eluted directly into an SW55 tube with 1.5 ml of 2mM EDTA, 0.1% SDS; the first two or three drops are discarded.

Eluted mRNA is precipitated by adding 1/10 vol. 3M NaOAc and filling the tube with EtOH. This is then mixed, chilled for 30 minutes at -20°C, and spun at 50k rpm at 5°C for 30 min. The EtOH is poured off and the tube is air dried. The mRNA pellet is resuspended in 50-100ul of RNase-free water. Approximately 5 ul is melted at 70° in MOPS/EDTA/formaldehyde and run on an RNase-free 1% agarose gel to check quality.

### cDNA Synthesis

From this, cDNA is synthesized. A preferred method of cDNA synthesis is a variant of that described by Gubler and Hoffman, (Gene 25:263-269 (1982)). This is carried out as follows:
a. First Strand. 4 ug of mRNA and heated to about 100°C in a microfuge tube for 30 seconds and quenched on ice. The volume is adjusted to 70 ul with RNase-free water. The following are added: 20 ul of RT1 buffer, 2 ul of RNAse inhibitor (Boehringer 36 u/ul), 1 ul of 5 ug/ul of oligo dT (Collaborative Research), 2.5 ul of 20 mM dXTP's (ultrapure), 1 ul of 1 M DTT and 4 ul of RT-LX (Life Science, 24 u/ul). The resulting combination is incubated at 42°C for 40 min. It is heated to inactivate (70°C 10 min).
b. Second Strand. 320 ul of RNAse free water, 80 ul of RT2 buffer, 5 ul of DNA Polymerase I (Boehringer, 5 U/ul), 2 ul RNAse H (BRL 2 u/ul). Incubate at 15°C for 1 hr and 22°C for 1 hr. Add 20 ul of 0.5M EDTA pH 8.0, phenol extract and EtOH precipitate by adding NaCl to 0.5M, linear polyacrylamide (carrier) to 20ug/ml, and filling tube with EtOH. Spin 2-3 minutes in microfuge, remove, vortex to dislodge precipitate high up on wall of tube, and respin 1 minute.
c. Adaptors. Resuspend precipitated cDNA in 240 ul of TE (10/1). Add 30 ul of 10x low salt buffer, 30ul of 10X low salt buffer, 30ul of 10X ligation additions, 3ul (2.4ug) of kinased 12-mer adaptor, 2ul (1.6ug) of kinased 8-mer adaptor, and 1 ul of T4 DNA ligase (BioLabs, 400 u/ul, or Boehringer, 1 Weiss unit/ml). Incubate at 15°C overnight. Phenol extract and EtOH precipitate as above (no extra carrier now needed), and resuspend in 100 ul of TE.

### Use of cDNA fragments in expression vectors

For use with the BstXI-based cDNA expression vectors of the invention, (see infra) oligonucleotide segments containing terminal sequences corresponding to BstXI sites on the vectors are ligated to the cDNA fragment desired to be inserted. The resulting fragments are pooled by fractionation. A preferred method is as follows:

Prepare a 20% KOA, 2mM EDTA, 1 ug/ml EthBr solution and a 5% KOAc, 2mM EDTA, 1 ug/mlg EthBr solution. Add 2.6 ml of 20% KOAc solution to back chamber of a small gradient maker. Remove air bubble from tube connecting the two chambers by allowing solution to flow into the front chamber and then tilt back. Close passage between chambers, and add 2.5ml. of the 5% solution to the front chamber. If there is liquid in the tubing from a previous run, allow the 5% solution to run just to the end of the tubing, and then return to chamber. Place the apparatus on a stirplate, set the stir bar moving as fast as possible, open the stopcock connecting the two chambers and then open the front stopcock. Fill a polyallomer SW55 tube from the bottom with the KOAc solution. Overlay the gradient with 100 ul of cDNA solution. Prepare a balance tube and spin the gradient for 3 hrs at 50k rpm at 22°C. To collect fractions from the gradient, pierce the SW55 tube with a butterfly infusion set (with the luer hub clipped off) close to the bottom of the tube and collect three 0.5ml fractions and then 6 0.25ml fractions into microfuge tubes (about 22 and 11 drops respectively). EtOH precipitate the fractions by adding linear polyacrylamide to 20 ug/ml and filling the tube to the top with EtOH. After cooling tubes, spin them in a microfuge for 3 min. Vortex and respin 1 min. Rinse pellets with 70% EtOH (respin). Do not dry to completion. Resuspend each 0.25ml fraction in 10 ul of TE. Run 1 ul on a 1% agarose minigel. Pool the first three fractions, and those of the last six which contain no material smaller than 1kb.

Suppressor tRNA plasmids may be propagated by known methods. In a preferred method according to the present invention, supF plasmids can be selected in nonsuppressing hosts containing a second plasmid, p3, which contains amber mutated ampicillin and tetracycline drug resistance elements (Seed, 1983). The p3 plasmid is derived from PR1, is 57kb in length, and is a stably maintained, single copy episome. The ampicillin resistance of this plasmid reverts at a high rate, so that amp^{r} plasmids usually cannot be used in p3-containing strains. Selection for tet resistance alone is almost as good as selection for ammp+tet resistance. However, spontaneous appearance of chromosomal suppressor tRNA mutations presents an unavoidable background (frequency about 10⁻⁹) in this system. Colonies arising from spontaneous suppressor mutations are usually bigger than colonies arising from plasmid transformation. Suppressor plasmids typically are selected for in LB medium containing amp at 12.5 ug/ml and tet at 7.5 ug/ml. For large plasmid preps, M9 casamino acids medium containing glycerol (0.8%) may be used as a carbon source, and the bacteria grown to saturation.

Vector DNA may be isolated by known methods. The following method is preferred for plasmid from 1 liter of saturated cells:

Spin down cells in 1 liter J6 bottles, 4.2k rpm, 25 minutes. Resuspend in 40 ml 10mM EDTA pH 8 (Thump on soft surface). Add 80 ml 0.2M NaOH, 1% SDS, swirl until clearish, viscous. Add 40 ml 5M KOAc, pH4.7 (2.5M KOAc, 2.5M HOAc) shake semi-vigorously (until lumps are 2-3 mm in size). Spin (same bottle) 4.2 rpm, 5 min. Pour supernatant through cheesecloth into 250 ml bottle. Fill bottle with isopropyl alcohol. Spin J6, 4.2k rpm, 5 min. Drain bottle, rinse gently with 70% EtOH (avoid fragmenting the pellet). Invert bottle, and remove traces of EtOH with Kimwipe. Resuspend in 3.5 ml Tris base/EDTA 20mM/10mM. Add 3.75 ml of resuspended pellet to 4.5g CsCl. Add 0.75 ml 10/mg/ml ethidium bromide, mix. Fill VTi80 tubes with solution. Run at a speed of 80 rpm for 2.5 hours or longer. Extract bands by visible light with 1 ml syringe and 20 gauge or lower needle. Cut top off tube, insert the needle upwards into the tube at an angle of about 30° with respect to the tube, (i.e., as shallowly as possible) at a position about 3mm beneath the band, with the bevel of the needle up. After the band is removed, pour tube contents into bleach. Deposit extracted bands in 13 ml Sarstedt tube. Fill tube to top with n-butanol saturated with 1M NaCl, extract. If a very large quantity of DNA is obtained, reextract. Aspirate butanol into trap containing 5M NaOH (to destroy ethidium). Add about equal volume 1M ammonium acetate to DNA (squirt bottle). Add about 2 volumes 95% ethanol (squirt bottle). Spin 10K rpm, 5 min. J2-21. Rinse pellet carefully with 70% ethanol. Dry with swab, or lyophilizer.

The vector may be prepared for cloning by known methods. A preferred method begins with cutting 20 ug of vector in a 200 ul reaction with 100 units of BstXI (New York Biolabs), cutting at 50°C overnight in a well-thermostatted water bath (i.e., circulating water bath). Prepare 2 KOAc 5-20% gradients in SW55 tubes as described above. Add 100 ul of the digested vector to each tube and run for 3 hrs, 50K rpm at 22°C. Examine the tube under 300nm UV light. The desired band will have migrated 2/3 of the length of the tube. Forward trailing of the band means the gradient is overloaded. Remove the band with a 1 ml syringe and 20 gauge needle. Add linear polyacrylamide and precipitate the plasmid by adding 3 volumes of EtOH. Resuspend in 50 ul of TE. Set up ligations using a constant amount of vector and increasing amounts of cDNAs. On the basis of these trial ligations, set up large scale ligation, which can be accomplished by known methods. Usually the entire cDNA prep requires 1-2 ug of cut vector.

Adaptors may be prepared by known methods, but it is preferred to resuspend crude adaptors at a concentration of 1 ug/ul, add MgSO₄ to 10 mM, and precipitate by adding 5 volumes of EtOH. Rinse with 70% EtOH and resuspend in TE at a concentration of 1 ug/ul. To kinase take 25ul of resuspended adaptors, add 3ul of 10X kinasing buffer and 20 units of kinase; incubate 37°C overnight.

Preparation of buffers mentioned in the above description of preferred methods according to the present invention will be evident to those of skill. For convenience, preferred buffer compositions are as follows:
- Loading Buffer:: 0.5 M LiCl, 10mM Tris pH 7.5, 1mM EDTA 0.1% SDS.
- Middle Wash Buffer: 0.15 M LiCl, 10mM Tris pH 7.5, 1mM EDTA 0.1% SDS.
- Rt1 Buffer:: 0.25 M Tris pH 8.8 (8.2 at 42°), 0.25 M KC1, 30 mM MgCl₂.
- RT2 Buffer:: 0.1 M Tris pH 7.5, 25 mM MgCl₂, 0.5 M KCl, 0.25 mg/ml BSA, 50 mM DTT.
- 10X Low Salt: 60 mM Tris pH 7.5, 60 mM MgCl₂, 50 mM NaCl, 2.5 mg/ml BSA 70 mM Me.
- 10X Ligation: 1mM ATP, 20 mM DTT, 1 mg/ml BSA 10 Additions: mM spermidine.
- 10X Kinasing: 0.5 M Tris pH 7.5, 10mM ATP, 20mM Buffer: DTT, 10 mM spermidine, 1 mg/ml BSA 100 mM MgCl₂.

By "vector" is meant a DNA molecule, derived from a plasmid or bacteriophage, into which fragments of DNA may be inserted or cloned. A vector will contain one or more unique restriction sites, and may be capable of autonomous replication in a defined host or vehicle organism such that the cloned sequence is reproducible. Thus, by "DNA expression vector" is meant any autonomous element capable of replicating in a host independently of the host's chromosome, after additional sequences of DNA have been incorporated into the autonomous element's genome. Such DNA expression vectors include bacterial plasmids and phages.

Preferred for the purposes of the present invention, however, are viral vectors, such as those derived from simian virus strain 40 (SV40). SV40 is a papovavirus having a molecular weight of 28 Mdal, and containing a circular double-stranded DNA molecule having a molecular weight of 3 Mdal, which comprises the entire genome of the virus. The entire nucleotide sequence of this single, small, covalently closed circular DNA molecule has been determined. Fiers et al., Nature 273:113-120 (1978); Reddy et al., Science 200:494-502 (1978). The viral DNA of SV40 may be obtained in large quantities, and the genomic regions responsible for various viral functions have been accurately located with respect to a detailed physical map of the DNA. Fiers et al., supra; Reddy et al., supra. The viral genome of SV40 can multiply vegetatively or as an integral part of cellular chromosomes, and a wealth of information exists on the replication and expression of this genome.

Also preferred for the purposes of the present invention is a single-stranded bacteriophage cloning vehicle, designated M13, having a closed circular DNA genome of approximately 6.5 kb. An advantage of utilizing M13 as a cloning vehicle is that the phage particles released from infected cells contain single-stranded DNA homologous to only one of the two complementary strands of the cloned DNA, which therefore can be used as a template for DNA sequencing analysis.

Even more preferred for the purposes of the present invention are the expression vectors designated piH3, piH3M, and CDM8, deposited at the American Type Culture Collection (ATCC), 12301 Parklawn Drive, Rockville, MD 20852, on February 24, 1988, in E. coli piH3 has accession number ATCC 67,634, piH3M has accession number ATCC 67,633 and CDM8 has accession number ATCC 67,635.

By "tissue culture" is meant the maintenance or growth of animal tissue cells in vitro so as to allow further differentiation and preservation of cell architecture or function or both. "Primary tissue cells" are those taken directly from a population consisting of cells of the same kind performing the same function in an organism. Treating such tissue cells with the proteolytic enzyme trypsin, for example, dissociates them into individual primary tissue cells that grow well seeded onto culture plates at high densities. Cell cultures arising from multiplication of primary cells in tissue culture are called "secondary cell cultures." Most secondary cells divide a finite number of times and then die. A few secondary cells, however, may pass through this "crisis period", after which they are able to multiply indefinitely to form a continuous "cell line." Cell lines often will contain extra chromosomes, and usually are abnormal in other respects as well. The immortality of these cells is a feature shared in common with cancer cells.

Preferred cell lines for use as tissue culture cells according to the present invention include the monkey kidney cell line, designated "COS." COS cells are those that have been transformed by SV40 DNA containing a functional early gene region but a defective origin of viral DNA replication. COS cell clone M6 is particularly preferred for use according to the method of the invention. Also preferred for the purposes of the present invention are murine "WOP" cells, which are NIH 3T3 cells transfected with polyoma origin deletion DNA. cDNA may be introduced into the host tissue culture cells of the present invention by any methods known to those of skill. Transfection may be accomplished by, for example, protoplast fusion by spheroplast fusion, or by the DEAE dextran method (Sussman et al., Cell. Biol. 4:1641-1643 (1984)).

If spheroplast fusion is employed, a preferred method is the following variant based on Sandri-Goldrin et al., Mol. Cell Bio. 1:743-752 (1981). Briefly, for example, a set of six fusions requires 100 ml of cells in broth. Grow cells containing amplifiable plasmid to OD600=0.5 in LB. Add spectinomycin to 100 ug/ml (or chloramphenicol to 150 ug/ml). Continue incubation at 37°C with shaking for 10-16 hours. (Cells begin to lyse with prolonged incubation in spectinomycin or chloramphenicol medium). Spin down 100 ml of culture (JA14/GSA rotor, 250ml bottle) 5 min. at 10,000 rpm. Drain well, resuspend pellet in bottle with 5ml cold 20% sucrose, 50mM Tris-HCL pH 8.0. Incubate on ice 5 min. Add 2 ml cold 0.25M EDTA pH 8.0, incubate 5 min. at 37°C (waterbath). Place on ice, check percent conversion to spheroplasts by microscopy. In flow hood, slowly add 20ml of cold DME/10% sucrose/10mM MgCl₂ (dropwise, ca. 2 drops per second). Remove media from cells plated the day before in 6cm dishes (50% confluent). Add 5ml of spheroplast suspension to each dish. Place dishes on top of tube carriers in swinging bucket centrifuge. Up to 6 dishes can be comfortably prepared at once. Dishes can be stacked on top of each other, but 3 in a stack is not advisable as the spheroplast layer on the top dish is often torn or detached after centrifugation. Spin at 1000xg 10 min. Force is calculated on the basis of the radius to the bottom plate. Aspirate fluid from dishes carefully. Pipet 1.5-2ml 50% (w/w) PEG1450 (or PEG1000)/50% DME (no serum) into the center of the dish. If necessary, sweep the pipet tip around to ensure that the PEG spreads evenly and radially across the whole dish. After PEG has been added to the last dish, prop all of the dishes up on their lids so that the PEG solution collects at the bottom. Aspirate the PEG. The thin layer of PEG that remains on the cells is sufficient to promote fusion; the layer remaining is easier to wash off, and better cell viability can be obtained, than if the bulk of the PEG is left behind. After 90 to 120 seconds (PEG 1000) or 120 to 150 seconds (PEG 1450) of contact with the PEG solution, pipet 1.5ml of DME (no serum) into the center of the dish. The PEG layer will be swept radially by the DME. Tilt the dishes and aspirate. Repeat the DME wash. Add 3ml of DME/10% serum containing 15 ug/ml gentamicin sulfate. Incubate 4-6 hours in incubator. Remove media and remaining bacterial suspension, add more media and incubate 2-3 days. Extensive washing of the cell layer to remove PEG tends to remove many of the cells without any substantial benefit. If the cells are allowed to sit in the second DME wash for a few minutes, most of the spheroplast layer will come up spontaneously; however it is preferred to wash briefly and allow the layer to come off in the complete medium at 37°C.

The PEG solution can be conveniently prepared by melting a fresh bottle of PEG at 60°C and pouring approximate 50 ml aliquots by means of a 50 ml centrifuge tube into preweighed bottles. The aliquoted PEG is stored at 5°C in the dark. To make up a fresh bottle, weigh the aliquot, remelt, and add an equal volume of DME (no serum). Adjust the pH with 7.5% Na Bicarbonate solution if necessary, and filter sterilize. The resulting PEG solution may be stored up to 3 months at room temperature without detectable adverse consequence.

Transfected host cells will be cultured according to the invention in order to accomplish expression of the protein encoded by the cDNA clone, and to increase the absolute numbers of cells available for subsequent immunoselection. Those skilled in the art will know of appropriate methods and media for this purpose, taking into account the cell type and other variables routinely considered. COS cells, for example, may be cultured in Dulbecco's modified Eagle's medium (DME) supplemented with 10% calf serum and gentamycin sulfate. Transient expression of transfected cells normally can be expected between 48 and 72 hours posttransfection. However, this time period may vary depending upon the type or strain of host cell used and the cell culture conditions, as will be apparent to those of ordinary skill.

Immunoprecipitation, blotting, and cDNA sequencing of genes cloned according to the methods of the present invention may be carried out by any convenient methods known to those of skill. For example, the immunoprecipitation protocol of Clark et al., Leukocyte Typing II, Vol. II, pp. 155-167 (1986), is preferred. Southern, Northern, or other blot analysis methods known to those of skill may be employed, using hybridization probes prepared by known methods, such as that of Hu et al. (Gene 18:271-277 (1982)). cDNA sequencing also may be accomplished by known methods, including the dideoxynucleotide method of Sanger et al., P.N.A.S. (USA) 74:5463-5467 (1977).

The antibodies used according to the present invention may be polyclonal or monoclonal. These may be used singly, or in conjunction with other polyclonal or monoclonal antibodies to effect immunoselection of cells expressing the desired antigen or antigens by the methods of the present invention. Methods of preparing antibodies or fragments thereof for use according to the present invention are known to those of skill.

Standard reference works setting forth general principles of immunology include Klein, J., Immunology: The Science of Self-Nonself Discrimination, John Wiley & Sons, publisher, New York (1982); Kennett, R., et al, eds., Laboratory Techniques in Biochemistry and Molecular Biology, Vol. 13, Elsevere, publisher, Amsterdam (1984).

The term "antibody" is meant to include the intact molecule as well as fragments thereof, such as, for example, Fab and F(ab)′₂ fragments, which also are able to bind to antigen. Polyclonal antibody preparations may be derived directly from the blood of the desired animal species after immunization with the antigen of interest, or a fragment thereof, using any of the standard protocols known to those of ordinary skill. Similarly, monoclonal antibodies may be prepared using known methods (Kohler et al., Eur. J. Immunol 6:292 (1976)). Use of monoclonal antibodies is preferred for the purposes of the present invention.

For the purposes of immunoselection according to the present invention, the tissue culture host cells which have been exposed to antibodies directed against the target cell surface antigen are separated from host cells which do not express the target antigen by distributing the cells onto a substrate coated with antibody directed against the antibody for the antigen. This technique, termed "panning," will be known to those of skill, and is described, for example, by Mage et al., J. Immunol. Meth. 15:47-56 (1977), and Wysocki and Sato, P.N.A.S. (USA) 75:2844-2848 (1978).

Panning according to the methods of the present invention may be carried out as follows:
a. Antibody-coated dishes. Bacteriological 60mm plates, Falcon 1007 or equivalent, or 10cm dishes such as Fisher 8-757-12 may be used. Sheep anti-mouse affinity purified antibody (from, for example, Cooper BioMedical (Cappell)) is diluted to 10ug/ml in 50mM Tris HCl, pH 9.5. Add 3ml per 6cm dish, or 10ml per 10cm dish. Let sit ca. 1.5 hrs., remove to next dish 1.5 hrs., then to 3rd dish. Wash plates 3x with 0.15 NaCl (a wash bottle is convenient for this), incubate with 3ml 1mg/ml BSA in PBS overnight, aspirate and freeze.
b. Panning. Cells will be in 60mm dishes. Aspirate medium from dish, add 2ml PBS/0.5mM EDTA/0.02% azide and incubate dishes at 37°C for 30 min. to detach cells from dish. Triturate cells vigorously with short pasteur pipet, and collect cells from each dish in a centrifuge tube. Spin 4 min. setting 2.5 (200 x g) (takes 5 min). Resuspend cells in 0.5 -1.) ml PBS/EDTA/azide/5% FBS and add antibodies. Incubate at least 30 min. on ice. Add an equal volume of PBS/EDTA/azide, layer carefully on 3 ml PBS/EDTA/azide/2% Ficoll, and spin 4 min. at setting 2.5. Aspirate supernatant in one smooth movement. Take up cells in 0.5ml PBS/EDTA/azide and add aliquots to antibody-coated dishes containing 3ml PBS/EDTA/azida/5% FBS by pipetting through 100 micron Nylon mesh (Tetko). Add cells from at most two 60mm dishes to one 60mm antibody-coated plate. Let sit at room temperature 1-3 hours. Remove excess cells not adhering to dish by gentle washing with PBS/5% serum or with medium. 2 or 3 washes of 3ml are usually sufficient.
c. Hirt Supernatant. A preferred variant of the method of Hirt, J. Molec. Biol. 26:365-369 (1967), is as follows: Add 0.4 ml 0.6% SDS, 10mM EDTA to panned plate. Let sit 20 minutes (can be as little as 1 min. if there are practically no cells on the plate). Pipet viscous mixture into microfuge tube. Add 0.1ml 5M NaCl, mix, put on ice at least 5 hrs. Keeping the mixture as cold as possible seems to improve the quality of the Hirt. Spin 4 min., remove supernatant carefully, phenol extract (twice if the first interface is not clean), add 10ug linear polyacrylamide (or other carrier), fill tube to top with EtOH, precipitate, and resuspend in 0.1ml. Add 3 volumes EtOH/NaOAc, reprecipitate and resuspend in 0.1 ml. Transform into MC1061/p3, preferably using the high efficiency protocol hereinafter described. If the DNA volume exceeds 2% of the competent cell aliquot, the transformation efficiency will suffer. 5% gives the same number of colonies as 2.5% (efficiency is halved).

It is preferred for this aspect of the present invention to use "blockers" in the incubation medium. Blockers assure that non-specific proteins, proteases, or antibodies present do not cross-link with or destroy the antibodies present on the substrate or on the host cell surface, to yield false positive or false negative results. Selection of blockers can substantially improve the specificity of the immunoselection step of the present invention. A number of non-specific monoclonal antibodies, for example, of the same class or subclass (isotype) as those used in the immunoselection step (e.g., IgG₁, IgG₂A, IgGm, etc.) can be used as blockers. Blocker concentration (normally 1-100ug/ul) is important to maintain the proper sensitivity yet inhibit unwanted interference. Those of skill also will recognize that the buffer system used for incubation may be selected to optimize blocking action and decrease non-specific binding.

A population of cells to be panned for those expressing the target cell surface antigen is first detached from its cell culture dish (harvested) without trypsin. The cells then are exposed to a first antibody, which may be polyclonal or monoclonal, directed against the antigen of interest or against a family of related antigens. At this initial stage, a single antibody or a group of antibodies may be used, the choice depending upon the nature of the target antigen, its anticipated frequency, and other variables that will be apparent to those of skill. Target antigens expressed on the surfaces of host cells will form an antigen-antibody complex.

The cells subsequently are placed in close apposition to a substrate, such as a culture dish, filter disc, or the like, which previously has been coated with a second antibody or group of antibodies. This second antibody will be directed against the first antibody, and its choice will be a matter of ordinary skill dictated by, for example, the animal in which the first antibody was raised. For example, if the first antibody was raised in mice, the second antibody might be directed against mouse immunoglobulins, raised in goats or sheep. Cells expressing the target antigen will adhere to the substrate via the complex formed between the antigen, the first antibody, and the second antibody. Adherent cells then may be separated from nonadherent cells by washing. DNA encoding the target antigen is prepared from adherent cells by known methods, such as that of Hirt, J. Molec. Biol. 26:365-369 (1967). This DNA may be transformed into E. coli or other suitable host cells for further rounds of fusion and selection, to achieve the desired degree of enrichment.

In the usual case, the initial rounds of immunoselection will employ a panel of first antibodies directed against an epitope or group of epitopes common to the family of antigens to which the target antigen belongs. This will be sufficient to narrow the number of clones for future rounds quite significantly. Two such rounds usually will be found adequate, but the number of rounds may vary as mentioned above. Thereafter, a single round of selection may be performed employing a single first antibody or a group of first antibodies recognizing only the target antigen.

By "substrate" is meant a solid surface to which antibodies may be bound for immunoselection according to the present invention. Known suitable substrates include glass, polystyrene, polypropylene, dextran, nylon, and other materials. Tubes, beads, microtiter plates, bacteriological culture dishes, and the like formed from or coated with such materials may be used. Antibodies may be covalently or physically bound to the substrate by known techniques, such as covalent bonding via an amide or ester linkage, or by absorption. Those skilled in the art will know many other suitable substrates and methods for immobilizing antibodies thereupon, or will be able to ascertain such substrates and methods using no more than routine experimentation.

The choice of host tissue culture cells for use according to the present invention preferably should be such as to avoid the situation in which the antibodies used for panning recognize determinants on untransfected cells. Thus, while COS cells are preferred for transient expression of certain surface antigens, more preferred are murine WOP cells. Of the latter, WOP 3027 cells are even more preferred. WOP cells allow virtually all antibodies to be used, since cross-reactions between murine antibodies and murine cell surface determinants are rare.

The insert size of the recombinant DNA molecule should be chosen to maximize the likelihood of obtaining an entire coding sequence. Those of skill will know various methods by which a preliminary determination of optimal insert size for a given gene may be determined.

### Vector construction and cDNA insertion

Vectors suitable for expression of cDNA in mammalian tissue culture cells may be constructed by known methods. Preferred for the purposes of the present invention is an expression vector containing the SV40 origin. The vector may contain a naturally derived or synthetic transcription origin, and the SV40 early region promoter. Even more preferred is a chimeric promoter composed of human cytomegalovirus immediate early enhancer sequences. Various "enhancer sequences" also may be used with SV40 vectors. These are described, for example, by Banerji et al., Cell 27:299-308 (1981); Levinson et al., Nature 295:568-572 (1982); and Conrad et al., Mol. Cell. Biol. 2:949-965 (1982).

Insertion of cDNA into the vectors of the present invention can occur, for example, by homopolymeric tailing with terminal transferase. However, homopolymeric tracts located 5′ to cDNA inserts may inhibit in vitro and in vivo expression. Thus, preferred for purposes of the present invention is the use of inverted identical cleavage sites separated by a short replaceable DNA segment. Such inverted identical cleavage sites, preferably employing the BstXI restriction endonuclease, may be used in parallel with cDNA synthetic oligonucleotides, giving the same terminii as the replaceable segment of the vector. In this manner, the cDNA cannot ligate to itself, but can ligate to the vector. This allows the most efficient use of both cDNA and vector.

Another embodiment of the present invention is the above-described efficient oligonucleotide-based strategy to promote cDNA insertion into the vector. The piH3M vector of the present invention is preferred, and employs the inverted endonuclease sites. This vector may contain an SV40 origin of replication, but a more preferred form contains an M13 origin. This vector, containing the M13 origin, allows high level expression in COS cells of coding sequences placed under its control. Also, the small size and particular arrangement of sequences in the plasmid permit high level replication in COS cells.

By "cell surface antigen" is meant any protein that is transported through the intracellular membrane system to the cell surface. Such antigens normally are anchored to the cell surface membrane through a carboxyl terminal domain containing hydrophobic amino acids that lie in the lipid bilayer of the membrane, and there exert their biological and antigenic effects. Antigens such as those of T-lymphocytes are particularly suited for gene cloning by the method of the present invention. However, cell surface antigens of any cells may be cloned according to the present method. Moreover, proteins not normally expressed on the cell surface may admit of cloning according to the present method by, for example, using fluorescence activated cell sorting (FACS) to enrich for fixed cells expressing intracellular antigens.

By "substantially pure" is meant any antigen of the present invention, or any gene encoding any such antigen, which is essentially free of other antigens or genes, respectively, or of other contaminants with which it might normally be found in nature, and as such exists in a form not found in nature. By "functional derivative" is meant the "fragments," "variants," "analogs," or "chemical derivatives" of a molecule. A "fragment" of a molecule, such as any of the antigens of the present invention, is meant to refer to any polypeptide subset of the molecule. A "variant" of such molecules is meant to refer to a naturally occurring molecule substantially similar to either the entire molecule, or a fragment thereof. An "analog" of a molecule is meant to refer to a non-natural molecule substantially similar to either the entire molecule or a fragment thereof.

A molecule is said to be "substantially similar" to another molecule if the sequence of amino acids in both molecules is substantially the same, and if both molecules possess a similar biological activity. Thus, provided that two molecules possess a similar activity, they are considered variants as that term is used herein even if one of the molecules contains additional amino acid residues not found in the other, or if the sequence of amino acid residues is not identical. As used herein, a molecule is said to be a "chemical derivative" of another molecule when it contains additional chemical moieties not normally a part of the molecule. Such moieties may improve the molecule"s solubility, absorption, biological half life, etc. The moieties may alternatively decrease the toxicity of the molecule, eliminate or attenuate any undesirable side effects of the molecule, etc. Moieties capable of mediating such effects are disclosed, for example, in Remington's Pharmaceutical Sciences, 16th ed., Mack Publishing Co., Easton, Penn. (1980).

Similarly, a "functional derivative" of a gene of any of the antigens of the present invention is meant to include "fragments," "variants," or "analogues" of the gene, which may be "substantially similar" in nucleotide sequence, and which encode a molecule possessing similar activity.

The substantially pure antigens that have been expressed by methods of the present invention may be used in immunodiagnostic assay methods well known to those of skill, including radio-immunoassays (RIAs), enzyme immunoassays (EIAs) and enzyme-linked immunosorbent assays (ELISAs). The substantially pure proteins of the present invention, in soluble form, may be administered alone or in combination with other antigens of the present invention, or with other agents, including lymphokines and monokines or drugs, for the treatment of immune-related diseases and disorders in animals, including humans. As examples of such disorders that may benefit from treatment with the substantially pure proteins of the present invention may be mentioned immune deficiency diseases, diseases of immediate type hypersensitivity, asthma, hypersensitivity pneumonitis, immune-complex disease, vasculitis, systemic lupus erythematosus, rheumatoid arthritis, immunopathogenic renal injury, acute and chronic inflammation, hemolytic anemias, platelet disorders, plasma and other cell neoplasms, amyloidosis, parasitic diseases, multiple sclerosis, Guillain-Barre syndrome, acute and subacute myopathic paralysis, myasthenia gravis, immune endocrinopathies, and tissue and organ transplant rejection, all as described in Petersdorf et al., eds., Harrison's Principles of Internal Medicine, supra. See also Weir, ed., supra; Boguslaski et al., eds., supra; and Holborow et al., eds., supra.

When used for immunotherapy, the antigens of the present invention may be unlabeled or labeled with a therapeutic agent. Examples of therapeutic agents which can be coupled to the antigens of the invention for immunotherapy are drugs, radioisotopes, lectins, and toxins.

The dose ranges for the administration of the antigens of the present invention are those large enough to produce the desired immunotherapeutic effect, but not so large as to cause adverse side effects, such as unwanted cross-reactions, anaphylactic reactions, and the like. Generally, the dosage employed will vary with the age, condition, sex, and extent of the disease in the patient. Counterindications (if any), immune tolerance and other variables also will affect the proper dosage. Administration may be parenteral, by injection or by gradual perfusion over time. Administration also may be intravenous, intraparenteral, intramuscular, subcutaneous, or intradermal.

Preparations for parenteral administration include sterile or aqueous or non-aqueous solutions, suspensions and emulsions. Examples of non-aqueous solvents include propylene glycol, polyethylene glycol, vegetable oils such as olive oil, and injectable organic esters such as ethyl oleate. Aqueous carriers include water, alcoholic and aqueous solutions, emulsions, or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives also may be present, such as, for example, antimicrobials, antioxidants, chelating agents, inert gases and the like. Such preparations, and the manner and method of making them, are known and described, for example, in Remington's Pharmaceutical Science, 16th ed., supra.

The antigens of the present invention also may be prepared as medicaments or pharmaceutical compositions comprising the antigens, either alone or in combination with other antigens or other agents such as lymphokines, monokines, and drugs, the medicaments being used for therapy of animal, including human, immune-related indications.

Although the antigens of the present invention may be administered alone, it is preferred that they be administered as a pharmaceutical composition. The compositions of the present invention comprise at least one antigen or its pharmaceutically acceptable salt, together with one or more acceptable carriers and optionally other therapeutic agents. By "acceptable" is meant that the agent or carrier be compatible with other ingredients of the composition and not injurious to the patient. Compositions include those suitable for oral, rectal, nasal, topical (including buccal and sublingual), vaginal, or parenteral administration. The compositions conveniently may be presented in unit dosage form, and may be prepared by methods well known in the pharmaceutical arts. Such methods include bringing into association the active ingredient with the carrier which constitutes one or more accessory ingredients. In general, compositions are prepared by uniformly and intimately bringing into association the active ingredient with liquid carriers or finely divided solid carriers, or both, and shaping the product formed thereby, if required.

Orally administered pharmaceutical compositions according to the present invention may be in any convenient form, including capsules, cachets, or tablets, each containing a predetermined amount of the active ingredient. Powders or granules also are possible, as well as solution or suspension in aqueous or nonaqueous liquids, or oil-in-water liquid emulsions, or water-in-oil liquid emulsions. The active ingredient also may be presented as a bolus, electuary or paste.

Having now described the invention, the same will be more fully understood by reference to the following examples, which are not intended in any way to limit the scope of the invention.

### EXAMPLE Isolation and Molecular Cloning of the Human CD40 Antigen

The rapid immunoselection cloning method of the present invention was applied to isolate and clone the CD40 antigen. The nucleotide sequence of CD40 is shown in Figure 17.

## Claims (Claims for the following Contracting State(s): AT, BE, CH, DE, FR, GB, GR, IT, LI, LU, NL, SE)

1. cDNA comprising the nucleotide sequence shown in Fig. 17 coding for CD 40.

2. An expression vector containing a cDNA according to claim 1.

3. A host cell containing an expression vector according to claim 2.

4. A method for producing CD 40 antigen containing the steps:
a) culturing a host cell according to claim 3; and
b) isolating said antigen.

## Claims (Claims for the following Contracting State(s): ES)

1. A method for producing CD 40 antigen encoded by a cDNA comprising the nucleotide sequence shown in Fig. 17 coding for CD 40 containing the following steps:
a) culturing a host cell containing an expression vector containing said cDNA; and
b) isolating said antigen.

## Patentansprüche (Patentansprüche für folgende(n) Vertragsstaat(en): AT, BE, CH, DE, FR, GB, GR, IT, LI, LU, NL, SE)

1. cDNA, enthaltend die in Fig.17 dargestellte Nucleotidsequenz, die für CD 40 codiert.

2. Expressionsvektor mit einem Gehalt an einer cDNA nach Anspruch 1.

3. Wirtszelle mit einem Gehalt an einem Expressionsvektor nach Anspruch 2.

4. Verfahren zur Herstellung von CD 40-Antigen, mit den Stufen:
a) Züchten einer Wirtszelle nach Anspruch 3; und
b) Isolieren dieses Antigens.

## Patentansprüche (Patentansprüche für folgende(n) Vertragsstaat(en): ES)

1. Verfahren zur Herstellung von CD 40-Antigen, codiert durch eine cDNA, enthaltend die in Fig. 17 dargestellte Nucleotidsequenz, die für CD 40 codiert, mit den folgenden Stufen:
a) Züchten einer Wirtszelle mit einem Gehalt an einem Expressionsvektor, der die cDNA enthält; und
b) Isolieren des Antigens.

## Revendications (Revendications pour l'(les) Etat(s) contractant(s) suivant(s): AT, BE, CH, DE, FR, GB, GR, IT, LI, LU, NL, SE)

1. ADNc comprenant la séquence nucléotidique représentée sur la figure 17 codant pour CD40.

2. Vecteur d'expression contenant un ADNc selon la figure 1.

3. Cellule hôte contenant un vecteur d'expression selon la revendication 2.

4. Procédé pour la production de l'antigène CD40, comportant les étapes suivantes:
a) culture d'une cellule hôte selon la revendication 3, et
b) isolement dudit antigène.

## Revendications (Revendications pour l'(les) Etat(s) contractant(s) suivant(s): ES)

1. Procédé pour la production de l'antigène CD40 codé par un ADNc comprenant la séquence nucléotidique représentée sur la figure 17, codant pour CD40, comportant les étapes suivantes:
a) culture d'une cellule hôte contenant un vecteur d'expression contenant ledit ADNc, et
b) isolement dudit antigène.
